# EUROPEAN PATENT SPECIFICATION

(11) **EP 2 291 149 B1**
(45) Date of publication and mention of the grant of the patent: **05.10.2011**
(21) Application number: 09707986.7
(22) Date of filing: 30.01.2009
(51) Int. Cl.: A61F 5/01

(54) **KNEE ERGONOMIC DEVICE FOR RESOLVING THE VERTICAL LOADS**
ERGONOMISCHE KNIE-VORRICHTUNG ZUR BEHEBUNG VON VERTIKALEN LASTEN
DISPOSITIF ERGONOMIQUE DE GENOU POUR RÉSOUDRE LES CHARGES VERTICALES

(30) Priority: 05.02.2008 IT TS20080001; 30.06.2008 IT GO20080002
(43) Date of publication of application: 09.03.2011
(73) Proprietor: Pellis, Giancarlo, 34141 Trieste (TS) (IT)
(72) Inventor: Pellis, Giancarlo, 34141 Trieste (TS) (IT)
(86) International application number: PCT/IT2009/000026
(87) International publication number: WO 2009/098724

(56) References cited:
- EP-A- 0 361 405
- WO-A-92/15264
- WO-A-97/38759
- DE-U1-202006 005 095
- US-A- 5 107 824
- US-A1- 2005 148 915

## Description

### Field of the invention

The device can be used in the medical sector as a knee brace supporting the vertical loads which are axially transmitted on cartilages.

### Background of the invention

The knee is the intermediate joint of the lower limb. Its degree of free range allows for a movement of flexo-extension.

The articular mechanics is complex and the type of movement performed is directly related to the opening angle of the knee. If we take the extended lower limb as a starting point, the leg carries out a motion of pure rotation over the first 25-30° of flexion. From that angle on, up to maximal flexion (around 135°), there is a double movement of rolling and gliding. More specifically, after an initial prevalence of rotation of the femoral condyles on the tibial tray, as the flexion movement proceeds, gliding becomes increasingly more progressive and predominant with respect to rolling. Such sliding motion of the joint is known as roto-traslatory.

Moreover, the different anatomical conformation of the lateral and medial compartments of the knee leads - during flexo-extension - to an automatic longitudinal rotation of the tibia vis-à-vis the femur, which is estimated at around 20 degrees by some authors.

Knee braces aim at containing the lateral movements of the knee after suffering a trauma.

Generally speaking, a common knee brace consists of a femoral support and a tibial support. The femoral support includes a lateral femoral arm and a medial femoral arm, which are secured to the thigh by means of straps, parallel to its longitudinal axis. The tibial support comprises a lateral tibial arm and a medial tibial arm, which are secured to the leg by means of straps, parallel to its longitudinal axis.

Each femoral arm is coupled with the respective tibial arm through a hinge at knee level which imposes its movement to the whole joint.

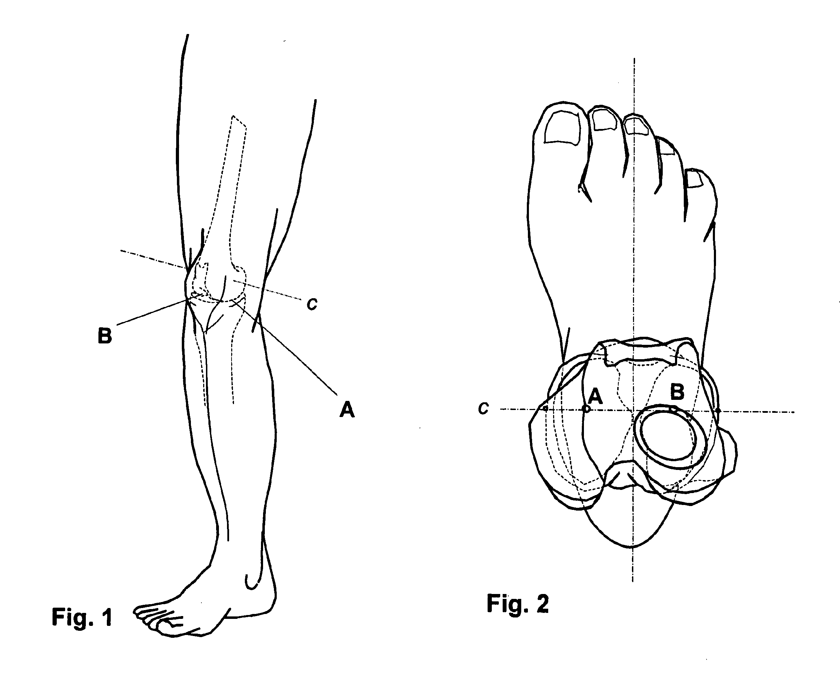

Looking at Figures 1 and 2, a more thorough analysis of the knee joint shows that, in the light of the rounded shape of the articular surfaces, there are only two contact points (covered with cartilage) in the joint: one is between the femoral condyle and the tibial tray in the medial compartment (point A), the other being between the femoral condyle and the tibial tray in the lateral compartment (point B).

A healthy knee, therefore, always preserves two contact points between the articular surfaces, their distances remaining perfectly constant.

When traumas and/or pathologies affect the proper functioning of an articular component, such as a ligament, a meniscus or a degenerated articular cartilage, the distance ratios between the articular surfaces are compromised and consequently prevent the entire knee from functioning properly. An injury to the cruciate ligaments does not allow for adequate control of the rolling and gliding movements of the femoral condyles on the tibial trays during flexo-extension. As a result, an anomalous friction of the articular surfaces takes place and the cartilages wear down.

All known devices impose their trajectory to the joint. If the trajectory does not coincide with the physiological one, a traditional device would drag the joint on its own trajectory thereby producing within the joint further tensions which are passed on to the organic structures already weakened by conditions and/or traumas.

This problem has been dealt with by various patents, including US Patent No. 5 107 824A, German Patent No. DE 20 2006 005095U1, US Patent 2005/148915 A1, European Patent No. 0 361 405, International Patent No. WO 84/03433, International Patent No. WO 92/15264 and International Patent No. WO 97/38759.

The hinge design for an articulating knee brace described in Pat. No. US 5 107 824A utilizes nested spherical hinge cups to mimic the movement of the human knee. More precisely a lateral hinge component is disposed on the lateral side of the wearer's knee and a medial hinge component is disposed on the medial side of the wearer's knee. The lateral hinge means has a first spherical shell connected to the linkage with thigh cuff, a second spherical shell is in nesting relationship with the first shell and connected to the linkage with calf cuff. The medial hinge means has a third spherical shell connected to the linkage with thigh cuff and a fourth spherical shell in nesting relationship with the third shell and connected to the linkage with calf cuff.

The lateral and medial hinges have respective first and second axes that are coincident when the leg is unflexed and in alignment with the transverse condylar axis of the leg. Control means permit to maintain the first axis in a relatively fixed position concentric with the transverse condylar axis of the knee and to permit movement of the second axis. The control means includes slots in the first shell that receive rivets fixedly secured to the second shell and second slots in the third shell that receive rivets fixedly secured to the fourth shell. More precisely the outer shell of lateral hinge is formed with a generally horizontal, slightly curved, slot and a generally vertical, curvilinear slot. The inner shell is formed with a pair of openings in which a pair of rivets is received. The rivets extend through slots in sliding engagement. The rivets secure outer shell in close sliding engagement with inner shell. The rivets can be formed with headed ends or can take the form of a headed nut and screw. The rivets are stationary with respect to inner shell. Outer shell moves relative to the rivets along a course defined by slots of the outer shell. The inner shell of the medial hinge is instead formed with a generally vertical, slightly curved, slot and a curvilinear slot.

The center of rotation of hinge member at any time is determined by the intersection of the normal lines to the slots at the points of rivets respectively. Given the changing degree of curvature of respective slots, the normal lines to these curves intersect at different points as flexion proceeds. Thus, although the centers of rotation of hinge members remain fixed, the center of rotation of the upper hinge members change continuously.

But the changing degree of curvature of internal curved slot determines a different vertical course (one longer and the other shorter) of the outer shell of lateral hinge and of the inner shell of medial hinge. The first and second thigh cuffs vertically slide and therefore they are not joined to the thigh.

The device described in Pat. No. DE 20 2006 005095 U1 is configured in the form of an exoprosthesis or orthosis for stabilizing a human knee and it includes a femur part associated with a thigh and configured to be affixed to the thigh, a tibia part associated with a lower leg and configured to be affixed to the lower leg, and a joint connecting the femur part to the tibia part. The joint has a sliding guide that is connected to the tibia part and that has groove-shaped recesses with which guide elements of the femur part engage in order to generate a multi joint motion. The joint is configured so that, during a flexing motion of the knee from a straight position to a flexed position, an instantaneous center of rotation of the joint moves along a curved centrode of the tibia having a vectorial vertical component and a vectorial sagittal component. The saggital component runs from posterior to anterior relative to the femur part whereas the vertical component runs from caudal to cranial relative to the femur part during the flexing motion of the lower leg. A first guide element moves into recesses parallel to the sagittal axis during the flexing motion, from a posterior position into an anterior position.

As above written, the leg carries out a motion of pure rotation over the first 25-30° of flexion. Instead the first guide element moves during this phase. Besides the first guide element moves toward the popliteus part of the knee during the first 90° of flexion and the second guide element moves back and downstairs. Over 90° the back positioning movement changes direction and the second guide element moves ahead. This movement produces a removal of the articular surfaces along the vectorial vertical component.

Pat. No. US 2005/148915 A1 disclosed is a knee brace that incorporates hinges that allow the brace to pivot simultaneously about a flexion-extension axis and a longitudinal rotation axis as the brace flexes and/or extends. The hinges include a medial hinge and a lateral hinge. The medial hinge defines a medial pivot axis and the lateral hinge defines a lateral pivot axis. The medial pivot axis does not coincide with the lateral pivot axis, and neither axis coincides with the flexion-extension axis. The brace comprises a rigid thigh frame, and a rigid calf frame. The thigh frame and calf frame are adapted to pivot relative to one another simultaneously about a substantially horizontal flexion-extension axis and a substantially vertical longitudinal rotation axis as the brace flexes and/or extends.

The calf portion includes an arcuately-shaped channel that is spaced from the pivot aperture. The channel extends along an arc of approximately 75°, and is located both superiorly and anteriorly of the pivot aperture. Two pivots are seat in the channel and in the aperture.

The hinges allow the brace to pivot and not to translate. The offset of the medial and lateral pivot axes mimes a translation but it does not permit the rotation of the calf frame.

A preferred embodiment describes that the calf portion includes a main plate portion that is pivotably secured to a superior end of the lateral upright on the calf frame. The projection is received within a substantially U-shaped recess in an inferior end of the main plate portion. The main plate portion is pivotable atop the lateral upright about a longitudinal axis of the upright. But the permitted rotation of the main plate portion does not correspond with the rotation of the calf portion which rotates around a vertical longitudinal tibial rotation axis.

The Patent No. WO 97/38759 describes a hinge characterized by a roto-traslatory motion. The hinge consists of two plates, which can freely rotate one on the other, each provided with a femoral arm and a tibial arm. The first plate has two openings, one placed centrally, the other peripherally. The central opening has a rectangular shape, with preferably rounded extremities. Its proximal extremity corresponds with a hole situated at the centre of the plate itself. The central opening starts from this central hole and proceeds towards the periphery of the plate along the "a" axis which coincides with the symmetry axis of the tibial arm. An extremity of this second opening is situated on a "b" axis, perpendicular to the "a" axis at a distance "l" from the centre of the above-mentioned central hole. The other extremity of the second opening is placed at 130-140 degrees vis-à-vis the axis which originates the first extremity. The peripheral opening is specifically shaped: initially, in the first 25-30 degrees starting from the above-mentioned axis which generates the first extremity, it is a circumference whose centre coincides with the centre of the plate, its radius being equal to "l"; subsequently, in the remaining 105-110 degrees, it is a spiral heading back towards the centre of the plate. The sequence of points which make up the longitudinal axis of the spiral is obtained from the sequence of points of the extremity of a segment having an "I" length, whose second extremity moves along the longitudinal axis of the central opening, from the centre of the plate to the outside.

The second plate has two pins, placed at a distance equal to "I", each with its longitudinal axis orthogonal to the rotation surfaces of the plates. The first pin is situated in a central position on the second plate and slides in the central opening of the first plate. The second pin is situated in a peripheral position and slides in the peripheral opening. They are both through pins and are equipped with a distant constraint that prevents disjunction of the two plates. The axis of the central pin is defined as "c" axis and corresponds with the horizontal intercondylar axis around which the initial rotation of the knee develops.

The resulting hinge performs a roto-traslatory motion. If we assume the extended leg as a starting point, in the first 25-30 degrees the hinge carries out a purely rotatory movement imposed by the second pin which slides in the circular section of the peripheral opening, similarly to the rotation described by the knee in this arch-shaped movement. As flexion proceeds, the rotatory motion is accompanied - in an increasingly progressive way - by a sliding motion of the second plate vis-à-vis the first one, when the second pin travels along the spiral-shaped part of the peripheral opening, similarly to what happens between the femoral condyles and the tibial trays in the 25-30° to 135° range.

The hinge described so far is functional for a theoretical knee. However, the length of the cruciate ligaments, their sizes and their insertion points - which characterize the articular surfaces - are different from one person to another. In order to study the true articular profile of a knee, it would be necessary to submit each individual to X-rays or to experimental measurements of the knee-malleolus distance; as a result, the spiral-shaped path followed by the hinge should be adjusted to that of the movement of the knee in each individual subject.

Patent No. WO 97/38759 does solve the problem of the typical roto-traslatory movement of the knee and is particularly indicated to restrain the knee laterally and antero-posteriorly in case of prevalently ligamentous deficiency. However, it does not take into account the longitudinal rotation of the tibia and the possibility of providing vertical support to the knee in case of traumas or degeneration of menisci and articular cartilages.

### Summary of the invention

The object of the present invention is to provide a knee device capable of tracking all the movements performed by the knee during flexo-extension. The device is also designed to break down the load determined by the bodyweight on various mechanical organs, parallel to the knee itself, so as to prevent the load itself from bearing on organic structures weakened by pathological and/or traumatic causes.

The device consists of a femoral support and a tibial support.

The femoral support comprises a lateral femoral arm and a medial femoral arm, parallel to one another and to the longitudinal axis of the thigh. They are joined together by means of a rigid or semirigid arch-shaped plate and straps passing through linear openings situated on the femoral arms. The femoral arms could also be joined together by straps alone.

The tibial support consists of a lateral tibial arm and a medial tibial arm, parallel to one another and to the longitudinal axis of the leg. They are joined together by means of straps which pass through linear openings situated on the tibial arms. The tibial arms could also be joined together by means of an arch-shaped plate and the afore-mentioned straps. Such tibial plate could also be secured to each tibial arm by a fastener.

The distal ends of the femoral arms and the proximal ends of the tibial arms are articulated to each other by means of at least two hinges, one placed laterally and the other medially to the knee. The distal ends of each femoral arm and the proximal ends of each tibial arm - which make up the hinge - are shaped as rounded plates.

The rounded distal end of each femoral arm accommodates four screwed pins and two openings. The first pin is placed centrally on the distal end along the "a" axis which coincides with the axis of longitudinal symmetry of the femoral arm itself. The second pin is placed peripherally on the end of the femoral arm, at a distance "l" with respect to the central pin, along a "b" axis perpendicular to the "a" axis. The axes "a" and "b" ideally divide the distal end of the femoral arm into four quadrants: the first one oriented towards the foot and the posterior part of the leg; the second one oriented towards the foot and the anterior part of the leg; the third one oriented towards the root of the lower limb and the posterior part of the leg; the fourth one oriented towards the root of the lower limb and the anterior part of the leg.

The third pin is placed proximally with respect to the afore-mentioned pins, in the quadrant oriented towards the root of the lower limb and the posterior part of the leg. It is situated at a distance "r" with respect to the central pin. The "d" axis which passes through the centre of the proximal pin and the centre of the central pin deviates by a few degrees from the "a" axis. The peripheral edge of the third pin is tangential to the "a" axis itself.

The "d" axis continues towards the peripheral distal edge of the femoral arm.

Along such axis - at a distance "r" from the central pin -is situated the fourth pin, diametrically opposed to the proximal pin vis-à-vis the central pin. The peripheral edge of the distal pin too is tangential to the "a" axis. The distal pin is situated in the quadrant oriented towards the foot and the anterior part of the leg.

The first opening is located near the peripheral edge of the rounded distal end of the femoral arm and is extended in the two quadrants oriented towards the posterior part of the leg. At the origin of this first opening, one of its extremities is situated at a distance "r" from the central pin along the "d1" axis which is perfectly symmetrical to the "d" axis vis-à-vis the "a" axis. The edge of the first extremity of the first opening is tangential to the "a" axis and to the peripheral edge of the distal pin. The first opening, after describing an initial arc of circumference, turns into a spiral which gets near the rounded distal end of the femoral arm.

The second opening, diametrically opposed to the first one with respect to the "a" axis, is located in the two quadrants oriented towards the anterior part of the leg. At the origin of this second opening, one of its extremities is situated at a distance "r" from the central pin and deviates by a few degrees from the longitudinal axis "a" of the femoral arm. The edge of the first extremity of the second opening is tangential to the "a" axis and to the peripheral edge of the proximal pin. The second opening, after describing an initial arc of circumference, turns into a spiral which - contrary to the first opening - gets away from the centre of the rounded distal end of the femoral arm.

The proximal end of each tibial arm is provided with three openings and two pins. The first tibial opening has a rectangular shape and rounded extremities. Its proximal extremity is obtained by creating a hole at the centre of the proximal extremity of each tibial arm and then proceeding towards the foot along the "a1" axis of longitudinal symmetry of the tibial arm.

The second opening is located peripherally on the proximal end of each tibial arm and is extended for 130°-140°. The first extremity of this peripheral opening is situated on a "b1" axis, perpendicular to the "a1" axis, at a distance "l" from the centre of the above-mentioned central hole from which the first opening originates. The other extremity of the second tibial opening is placed at 130-140° with respect to the axis which originates the first extremity. The peripheral opening, after describing an initial circumference whose centre coincides with that of the proximal extremity of the central opening and whose radius is equal to "I", turns into a spiral which returns towards the centre of the plate.

The "a1" and "b1" axes ideally divide the proximal end of each tibial arm into four quadrants; the first one is oriented towards the foot and the posterior part of the leg; the second one is oriented towards the foot and the anterior part of the leg; the third one is oriented towards the root of the lower limb and the posterior part of the leg; the fourth one is oriented towards the root of the lower limb and the anterior part of the leg. The central opening is placed in the two quadrants oriented towards the foot, the second tibial opening is placed in the two quadrants oriented towards the root of the lower limb.

The third tibial opening, which is extended for 130°-140°, has a first semicircular extremity that is placed at a distance "r" from the intersection of the "a1" and "b1" axes in the quadrant oriented towards the foot and the anterior part of the leg, and deviates by a few degrees from the "a1" axis. The edge of the extremity of the third tibial opening is tangential to the "a1" axis. The third tibial opening, after describing an initial arc of circumference, turns into a spiral which gets near the rounded proximal end of the tibial arm.

The first tibial pin is situated distally vis-à-vis the central opening, at a distance "r" from the intersection of the "a1", "b1" axes, in the quadrant oriented towards the foot and the posterior part of the leg. The "d2" axis which passes through the centre of the pin and the intersection of the "a1", "b1" axes deviates by a few degrees from the "a1" axis. Its peripheral edge is tangential to the "a1" axis itself and to the first extremity of the second peripheral tibial opening.

The second tibial pin is diametrically opposed to the first tibial pin with respect to the "a1" axis; consequently the axis which passes through the centre of the pin itself and the intersection of the "a1" and "b1" axes deviates by a few degrees from the "a1" axis, though in the opposite direction. The peripheral edge of the second tibial pin too is tangential to the "a1" axis. The second tibial pin is placed in the quadrant oriented towards the root of the lower limb and the anterior part of the leg.

The peripheral edge of the end of the tibial arm is extended in the quadrants oriented towards the posterior part of the leg and is shaped in such a particular way that its initial section - extending in the posterior quadrant oriented towards the root of the limb - describes a circumference arc. The successive section of the peripheral edge - extending in the posterior quadrant oriented towards the foot - is shaped as a spiral that tends to get away from the centre of the plate. The peripheral surface of the proximal pin of the femoral arm remains in constant contact with the peripheral edge of the tibial arm during the roto-traslatory movement of the latter vis-à-vis the femoral arm.

The longitudinal axis of the central pin of the lateral hinge and the longitudinal axis of the central pin of the medial hinge are coaxial. Such axis coincides with the "c" axis which passes through the femoral condyles and around which the phase of initial rotation of the knee takes place.

The medial tibial arm can be divided in two parts: a fixed medial tibial arm (fastened to a tibial plate) and a free-moving medial tibial arm which pivots on the fixed arm itself by means of a second medial hinge. The tibial plate is arc-shaped, is made of rigid material with a good harmonic response and tracks the anterior part of the leg. The mobile medial tibial arm consists of a rectilinear plate extending between the medial femoral arm and the fixed medial tibial arm. The medial femoral arm of the femoral support is secured to the proximal end of the free-moving medial tibial arm by means of the above-mentioned medial hinge. The distal end of the mobile medial arm is provided with a hole (situated along the symmetry axis of the mobile arm itself), engaged by a pin placed at the intersection between the symmetry axis of the tibial plate and the symmetry axis of the fixed medial tibial arm. An arc-shaped opening is located along the symmetry axis of the mobile medial tibial arm, in a proximal position with respect to the hole. Such opening accommodates a second pin, which is secured to the fixed medial tibial arm and is situated proximally vis-à-vis the pin along the symmetry axis of the fixed medial tibial arm itself. These elements form a second medial hinge.

More arc-shaped openings and more holes can be made on the distal end of the free-moving medial tibial arm along its symmetry axis. The centre of a semicircular extremity of the openings is placed along the same symmetry axis.

The lateral tibial arm too can be divided into a proximal lateral tibial arm and a distal lateral tibial arm. The proximal lateral tibial arm has an elongated shape and the distal lateral tibial arm is fastened to the tibial plate. At least three equidistant holes are made on the prolongation of the proximal lateral tibial arm and along its symmetry axis. The distal lateral tibial arm accommodates at least two threaded holes, coaxial to at least two holes present on the proximal lateral tibial arm. Two screws pass through the holes of the proximal lateral tibial arm and are secured to two threaded holes of the distal lateral tibial arm.

The pins of the distal ends of the femoral arms and of the fixed medial tibial arm are secured to a plate external to each hinge by means of fasteners. In each plate there are holes placed coaxially with the pins of the femoral arms and of the fixed medial tibial arm. It is through such holes of each plate that the free ends of the pins themselves or the afore-mentioned fasteners engaged to the pins do pass, thereby securing each plate to each femoral arm and to the fixed medial tibial arm, and preventing their disjunction. The fasteners could be screws for engaging the threaded holes of the pins or rivets with their free end engaging in the holes of the pins or nuts that engage the external thread of the pins. The height of the pins is greater than the thickness of the tibial arm.

The present device is capable - even in weight-bearing conditions - of keeping the articulation surfaces well spaced, though submitted to the weight of the body that tends to compress them on one another.

The knee support, while tracking the knee in its motion of antero-posterior flexo-extension, at the same time prevents the lateral and antero-posterior movements of the knee determined by deficient articular structures following a trauma.

The device allows the knee to carry out an ergonomic movement which adequately tracks the physiological one, particularly in the light of the longitudinal automatic rotation of the tibia, contrary to the movement imposed by the best known devices.

### Brief description of the drawings

Further features of the present invention will emerge from the description of an embodiment, preferable but not exclusive, of the device, illustrated in the attached drawings by way of example and not limitation:
- Figure 1 is a front elevation view of a lower limb;
- Figure 2 is an elevation view from above of the tibial trays along the transversal plane of the knee, showing the points of contact with the femoral condyles and the "c" axis;
- Figure 3 is a front elevation view of the knee;
- Figure 4 is an elevation view from above of the tibial trays along the transversal plane of the knee, showing the points of contact with the femoral condyles;
- Figure 5 shows the lower limb in extended position with the indication of the longitudinal axis, transversal axis and front plane;
- Figure 6 is an elevation view from above of the skeletal portion of the knee, showing the points of contact between the articulation surfaces of the knee and the front elevation view;
- Figure 7 shows the lower limb in flexed position with the indication of the front plane of the thigh and the front plane of the leg;
- Figure 8 is an elevation view from above of the skeletal portion of the knee flexed at 90 degrees, showing the contact points between the articulation surfaces of the knee, the front elevation view of the leg and the front elevation view of the thigh;

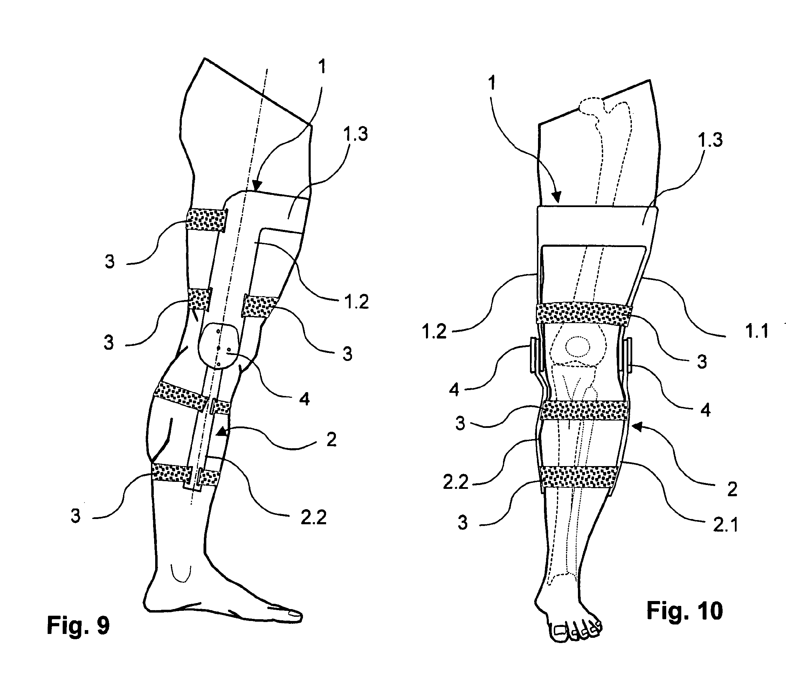

- Figure 9 is a lateral elevation view of a first preferred embodiment of the present invention, secured to the wearer's limb from a medial perspective;
- Figure 10 is a front elevation view of the device shown in Figure 9, secured to the wearer's limb viewed frontally;

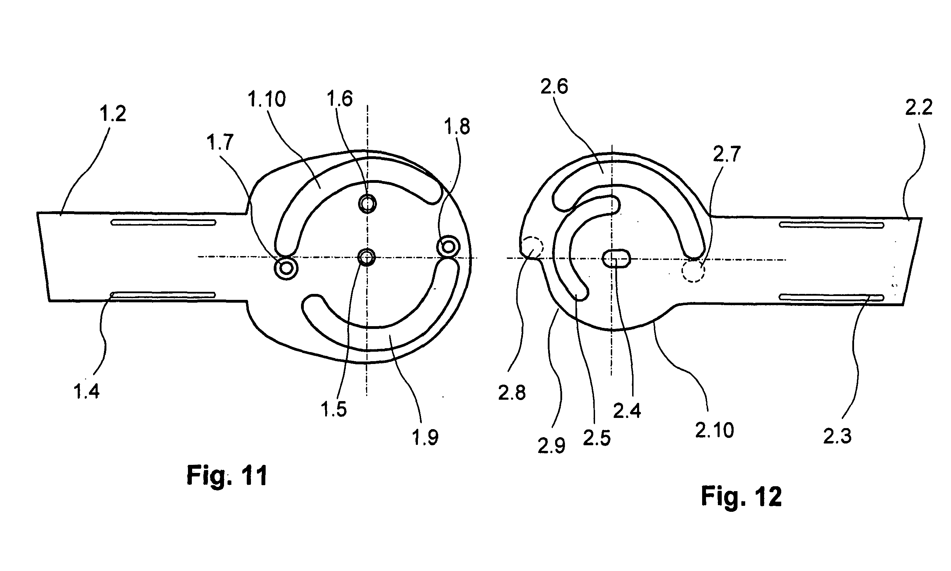

- Figure 11 is a lateral elevation view of a femoral arm of the device;
- Figure 12 is a lateral elevation view of a tibial arm of the device;

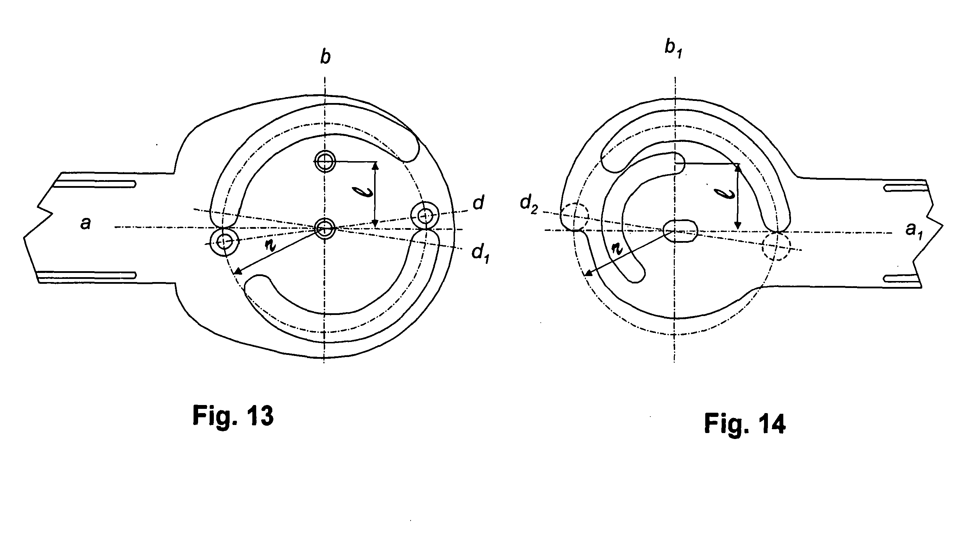

- Figure 13 is a lateral elevation view of the same femoral arm shown in Figure 11 with some schematic representations of construction;
- Figure 14 is a lateral elevation view of the same tibial arm shown in Figure 12 with some schematic representations of construction;

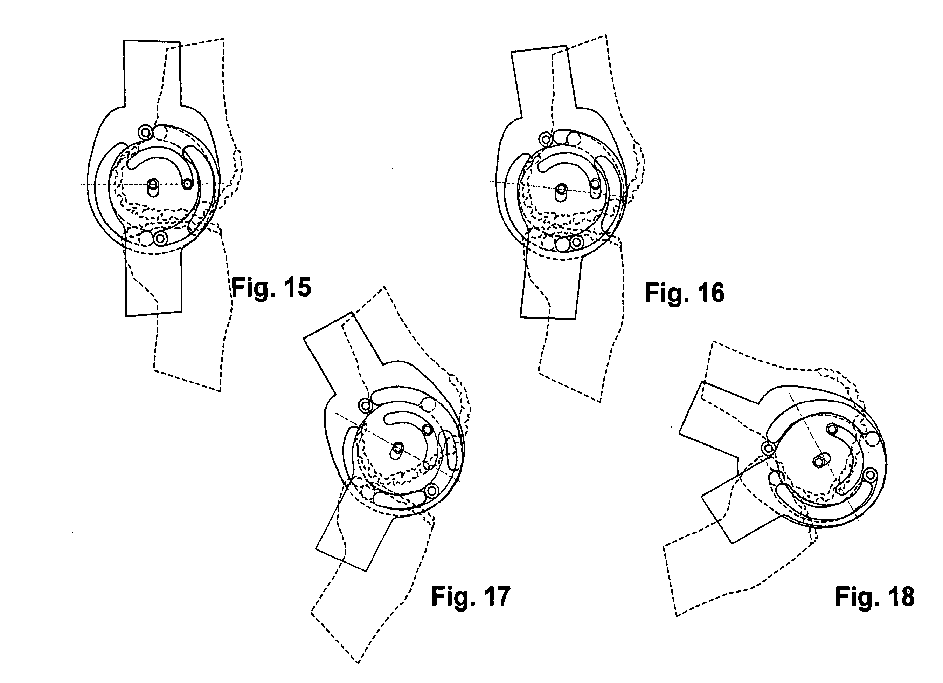

- Figures 15, 16, 17 and 18 show the movements of a hinge of the device;

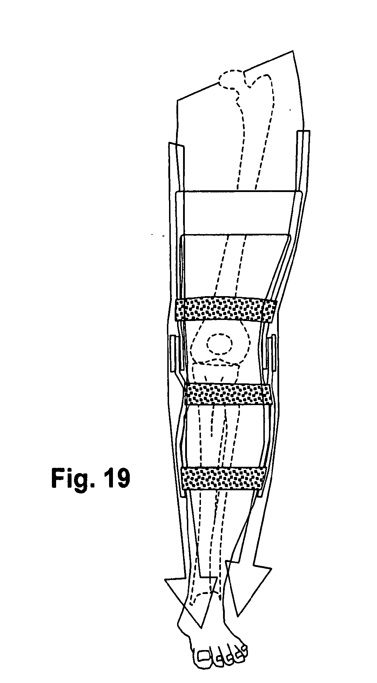

- Figure 19 shows the distribution of load on the leg when using the present device;

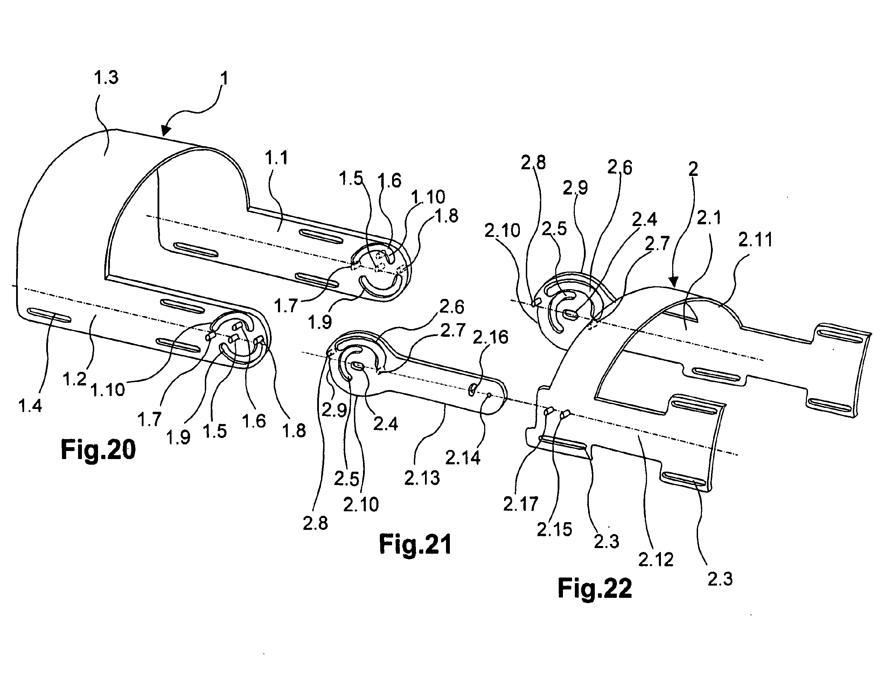

- Figure 20 is a perspective view of the femoral support of the device in a second preferred embodiment;
- Figure 21 is a perspective view of the free-moving medial arm of the device in a second preferred embodiment;
- Figure 22 is a perspective view of the tibial support of the device in a second preferred embodiment;

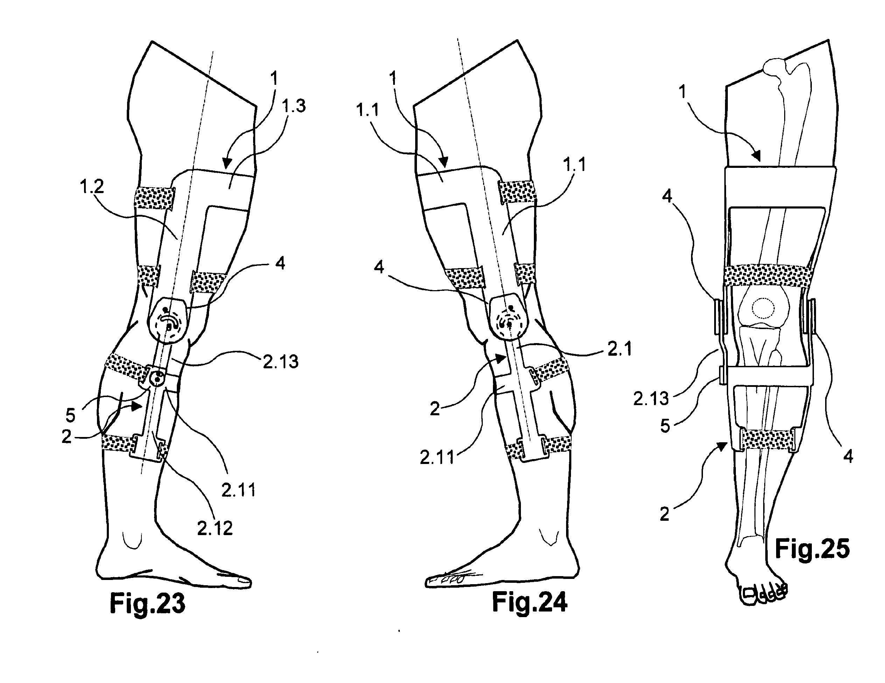

- Figure 23 is a lateral elevation view of the device in a second preferred embodiment, secured to the wearer's limb viewed medially;
- Figure 24 is a lateral elevation view of the device in a second preferred embodiment, secured to the wearer's limb viewed laterally;
- Figure 25 is a front elevation view of the device in a second preferred embodiment, secured to the wearer's limb viewed frontally;

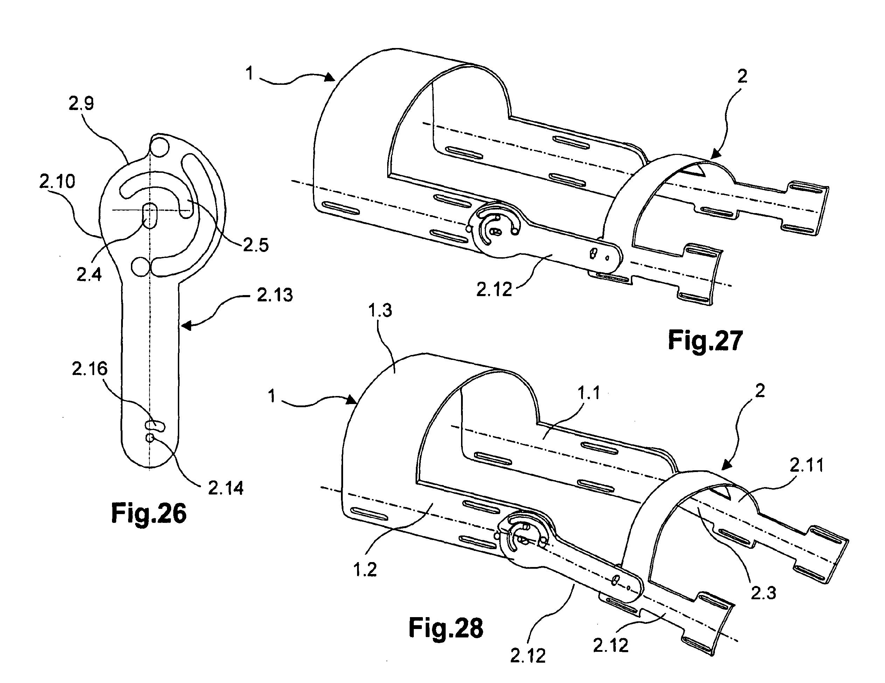

- Figure 26 is a front elevation view of the free-moving tibial arm;
- Figure 27 shows the device in a second preferred embodiment, without the covering plates, in a position corresponding to that adopted when secured to the leg in the extended position;
- Figure 28 shows the same device, without the covering plates, in a position corresponding to that adopted when secured to the leg on a 15-degree flexion;

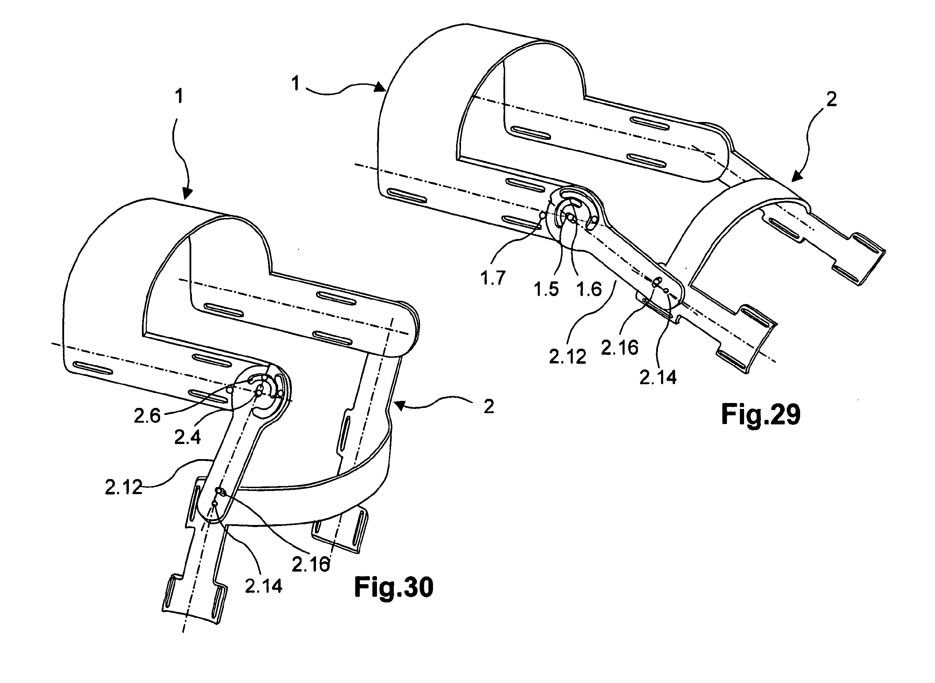

- Figure 29 shows the same device, without the covering plates, in a position corresponding to that adopted when secured to the leg on a 35-degree flexion;
- Figure 30 shows the same device, without the covering plates, in a position corresponding to that adopted when secured to the leg flexed above 90 degrees;

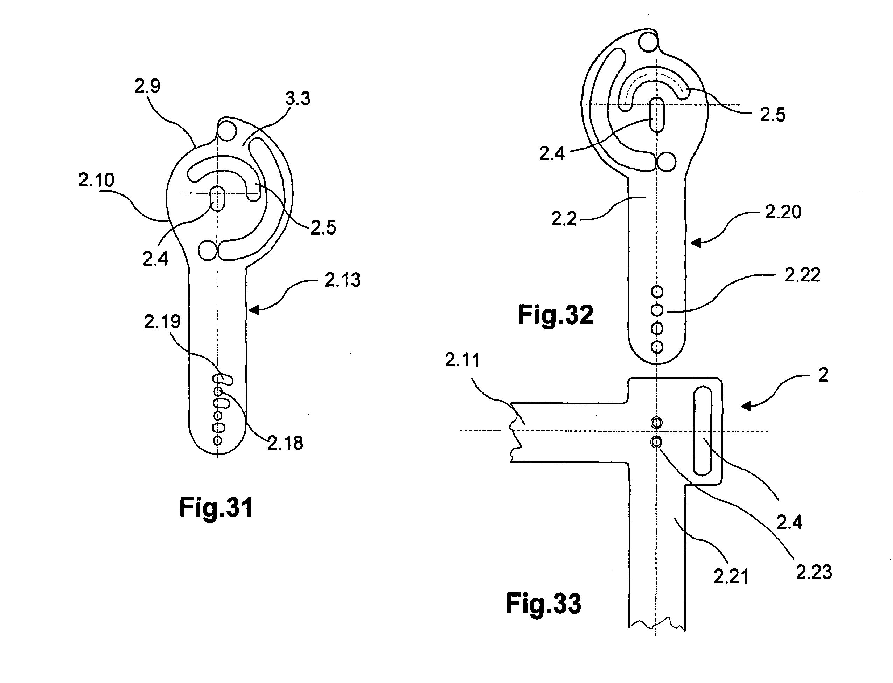

- Figure 31 is a front elevation view of a modified free-moving tibial arm;
- Figure 32 is a lateral elevation view of a modified proximal lateral tibial arm, separated from the distal lateral tibial arm;
- Figure 33 is a lateral elevation view of a modified distal lateral tibial arm;

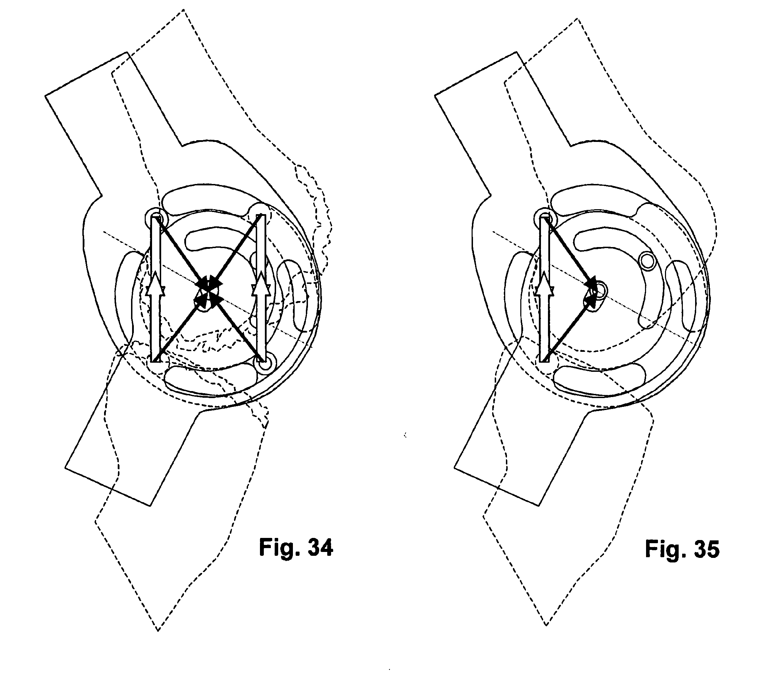

- Figure 34 shows the distribution of forces on a hinge in case of chondropathy;
- Figure 35 shows the distribution of forces on a hinge in case of injury or trauma to the posterior cruciate ligament.

### Detailed description of two preferred embodiments

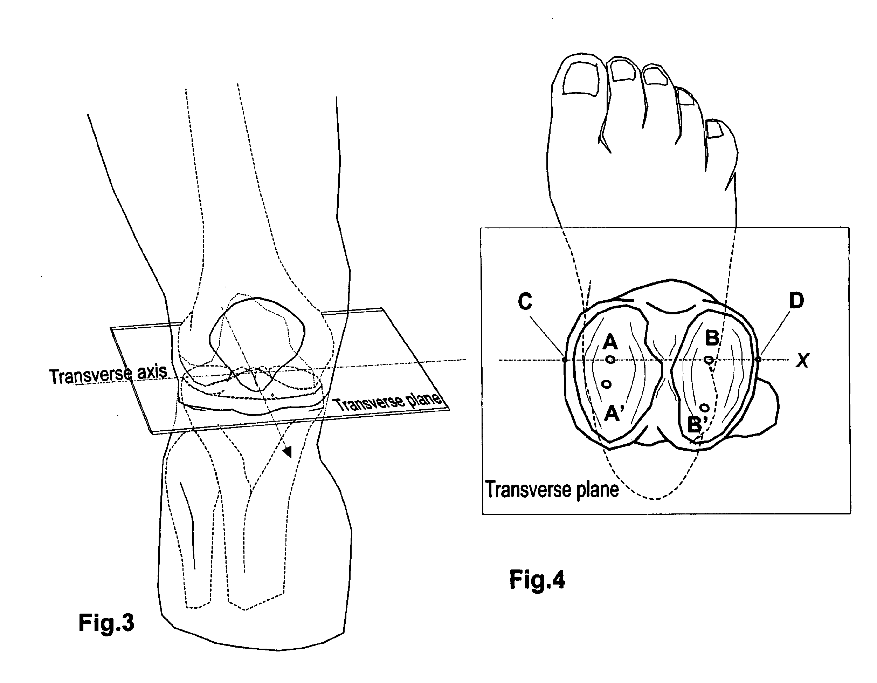

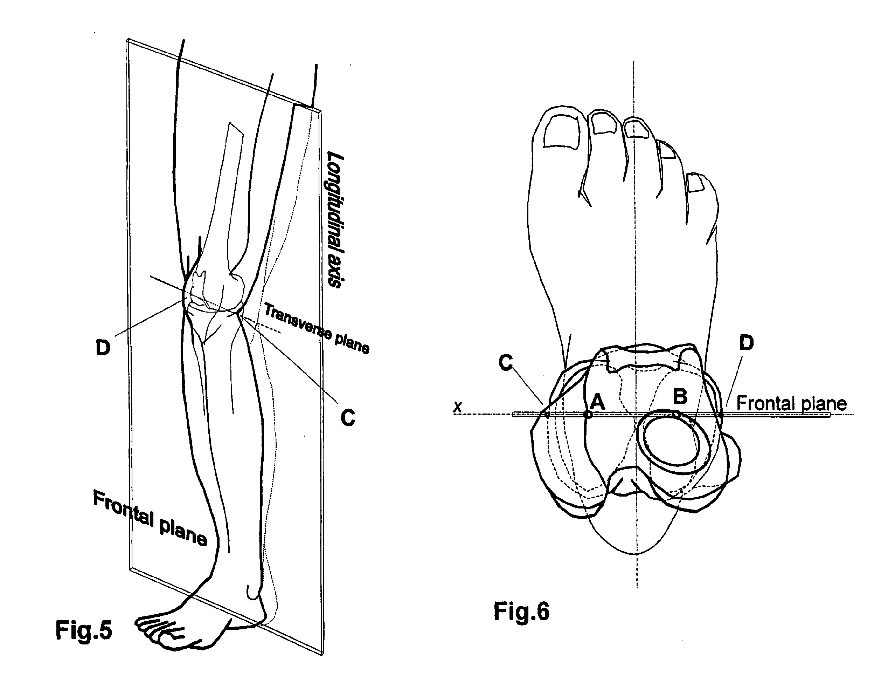

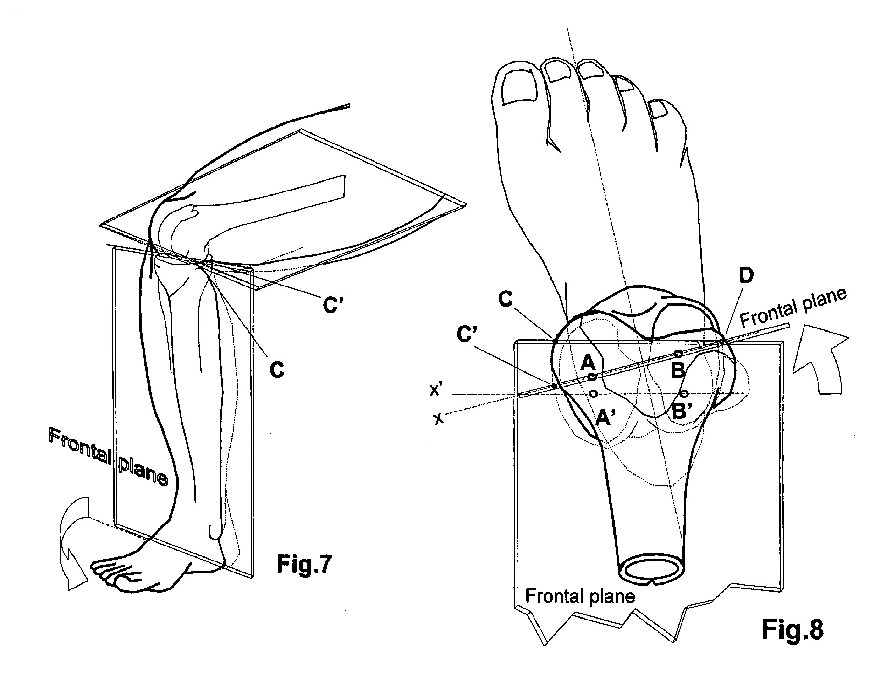

As shown in Figures 3 to 8, the first preferred embodiment of the present invention consists of a femoral support 1 and a tibial support 2.

The femoral support 1 comprises a lateral femoral arm 1.1 and a medial femoral arm 1.2, which are parallel to each other and to the longitudinal axis of the thigh and are joined together by means of an arch-shaped rigid or semirigid plate 1.3 and by straps 3.

The inner surface of the femoral plate 1.3 tracks the anterior part of the leg.

Linear openings 1.4 are present on each femoral arm 1.1, 1.2. The straps 3, which have a face made of nylon Velcro^{®}, pass through such linear openings 1.4. The straps are wrapped all around the thigh anteriorly and posteriorly, thus securing the femoral support 1.1 to the thigh, together with the plate 1.3.

The tibial support 2 consists of a lateral tibial arm 2.1 and a medial tibial arm 2.2, which are parallel to each other and to the longitudinal axis of the leg. Linear openings 2.3 are present on the tibial arms 2.1, 2.2 too. The straps 3, which have a face made of nylon Velcro^{®}, pass through such linear openings 2.3, and are wrapped all around the leg, thus securing the tibial support 2 to the leg itself.

The distal ends of the femoral arms 1.1, 1.2 and the proximal ends of the tibial arms 2.1, 2.2 are joined together by means of two hinges, one placed laterally with respect to the knee, the other placed medially. The distal ends of each femoral arm 1.1, 1.2 and the proximal ends of the each tibial arm 2.1, 2.2, which make up the hinge, are shaped as plates and are rounded.

For clarity purposes, let us consider only the hinge present in the medial compartment of the knee: four pins 1.5, 1.6, 1.7, 1.8 are fixed on the rounded distal end of the medial femoral arm 1.2; two openings 1.9, 1.10 are also present on the same end of the medial femoral arm 1.2.

A first pin 1.5 is located on the distal end of the medial femoral arm 1.2 and is placed centrally along the "a" axis which coincides with the axis of longitudinal symmetry of the medial femoral arm 1.2 itself.

A second pin 1.6 is located on the distal end of the medial femoral arm 1.2 and is placed peripherally at a distance "l" with respect to the central pin 1.5, along the "b" axis, perpendicular to the "a" axis.

The axes "a" and "b" ideally divide the distal end of the medial femoral arm 1.2 into four quadrants: the first one oriented towards the foot and the posterior part of the leg; the second one oriented towards the foot and the anterior part of the leg; the third one oriented towards the root of the lower limb and the posterior part of the leg; the fourth one oriented towards the root of the lower limb and the anterior part of the leg.

The third pin 1.7 is placed proximally with respect to the afore-mentioned pins 1.5, 1.6, in the quadrant oriented towards the root of the lower limb and the posterior part of the leg. It is situated at a distance "r" with respect to the central pin 1.5. The "d" axis which passes through the centre of the proximal pin 1.7 and the centre of the central pin 1.5 deviates by a few degrees from the "a" axis. The peripheral edge of the proximal pin 1.7 is tangential to the "a" axis itself.

The "d" axis, which passes through the centre of the proximal pin 1.7 and the centre of the central pin 1.5, continues its path towards the peripheral distal edge of the medial femoral arm 1.2. Along such axis - at a distance "r" from the central pin 1.5 - there is a fourth pin 1.8, diametrically opposed to the pin 1.7 vis-à-vis the central pin 1.5. The peripheral edge of the distal pin 1.8 too is tangential to the "a" axis. The distal pin 1.8 is situated in the quadrant oriented towards the foot and the anterior part of the leg.

The first opening 1.9 is located near the peripheral edge of the rounded distal end of the medial femoral arm 1.2 and is extended in the two quadrants oriented towards the posterior part of the leg for 130-140 degrees. The centre of a first semicircular extremity of the opening 1.9 is situated at a distance "r" from the central pin 1.5 along a "d1" axis which is perfectly symmetrical to the "d" axis vis-à-vis the "a" axis. The edge of the first semicircular extremity of the opening 1.9 is tangential to the "a" axis and to the peripheral edge of the distal pin 1.8. The opening 1.9 has a particular shape: over the first 25-30 degrees starting from the first extremity, it describes an arc of circumference; from 25-30 degrees on, up to 130-140 degrees, it turns into a spiral which gets near the rounded distal end of the medial femoral arm 1.2. More specifically, the points that make up the longitudinal axis of this spiral are situated at an increasingly lower distance from the centre of the rounded distal end of the medial femoral arm 1.2 as they get closer to the second extremity, which is semicircular too.

The second opening 1.10, diametrically opposed to the opening 1.9 with respect to the "a" axis, is located in the two quadrants oriented towards the anterior part of the leg. The centre of a first semicircular extremity of the opening 1.10 is situated at a distance "r" from the central pin 1.5 along the "d1" axis. The edge of the first semicircular extremity of the opening 1.10 is tangential to the "a" axis and to the peripheral edge of the proximal pin 1.7.

The opening 1.10 too describes an arc of circumference over the first 25-30 degrees starting from the first extremity. Yet - contrary to the opening 1.9 - from 25-30 degrees on, up to 130-140 degrees, it turns into a spiral which gets away from the centre of the rounded distal end of the medial femoral arm 1.2. More specifically, the points that make up the longitudinal axis of this spiral are situated at an increasingly greater distance from the centre of the rounded distal end as they get closer to the second extremity.

The plate located at the proximal end of the medial tibial arm 2.2 is provided with three openings 2.4, 2.5, 2.6 and two pins 2.7, 2.8.

The first tibial opening 2.4 has a rectangular shape and rounded extremities.

The first tibial opening 2.4 is obtained by creating a hole at the centre of the plate of the proximal end of the medial tibial arm 2.2 and then proceeding towards the foot along the "a1" axis of longitudinal symmetry of the medial tibial arm 2.2. The hole is the proximal extremity of the first tibial opening 2.4.

The second opening 2.5 is located peripherally on the proximal end of the medial tibial arm 2.2 and is extended for 130°-140°. The first extremity of this second opening 2.5 is situated on a "b1" axis, perpendicular to the "a1" axis, at a distance "l" from the centre of the above-mentioned central hole from which the first opening 2.4 originates towards the anterior part of the leg. The other extremity of the peripheral opening 2.5 is placed at 130-140 degrees with respect to the "b1" axis. The peripheral opening 2.5 has a particular shape: over the first 25-30 degrees starting from the afore-mentioned "b1" axis, it describes a circumference whose centre coincides with that of the hole generating the central opening and whose radius is equal to "l".

Subsequently, for the remaining 105-110 degrees, it turns into a spiral which returns towards the centre of the plate. The longitudinal axis of such spiral is obtained as the sequence of points of the end of a segment with a length "l" whose second end proceeds along the longitudinal axis of the central opening 2.4 (from the centre of the plate to the periphery).

The "a1" and "b1" axes ideally divide the proximal end of each medial tibial arm 2.2 into four quadrants; the first one is oriented towards the foot and the posterior part of the leg; the second one is oriented towards the foot and the anterior part of the leg; the third one is oriented towards the root of the lower limb and the posterior part of the leg; the fourth one is oriented towards the root of the lower limb and the anterior part of the leg.

The central opening 2.4 is placed in the two quadrants oriented towards the foot. The first peripheral opening 2.5 is placed in the two quadrants oriented towards the root of the lower limb.

The centre of a first semicircular extremity of the third opening 2.6, which is extended for 130-140 degrees, is placed at a distance "r" from the intersection of the "a1", "b1" axes in the quadrant oriented towards the foot and the anterior part of the leg, and deviates by a few degrees from the "a1" axis. The edge of the extremity of the opening 2.6 is tangential to the "a1" axis. Over the first 25-30 degrees, the opening 2.6 describes an arc of circumference, while from 25-30 degrees up to 130-140 degrees, it turns into a spiral which gets near the centre of the rounded proximal end of the medial tibial arm 2.2. More specifically, the points that make up the longitudinal axis of such spiral are at an increasingly lower distance from the centre of the rounded proximal end as they get near the second extremity.

The first tibial pin 2.7 is situated distally vis-à-vis the central opening 2.4, at a distance "r" from the intersection of the "a1", "b1" axes, in the quadrant oriented towards the foot and the posterior part of the leg. The "d2" axis which passes through the centre of the pin 2.7 and the intersection of the "a1", "b1" axes deviates by a few degrees from the "a1" axis. The peripheral edge of the first tibial pin 2.7 is tangential to the "a1" axis and to the first extremity of the second peripheral opening 2.6.

This "d2" axis continues towards the proximal peripheral edge of the medial tibial arm 2.2. The second pin 2.8 is placed on such axis, at a distance "r" with respect to the intersection of the "a1", "b1" axes. The pin 2.8 is diametrically opposed to the pin 2.7 with respect to the "a1" axis; consequently the "d2" axis which passes through the centre of the pin 2.8 and the intersection of the "a1", "b1" axes deviates by a few degrees from the "a1" axis, though in the opposite direction. The peripheral edge of the pin 2.8 too is tangential to the "a1" axis. The pin 2.8 is placed in the quadrant oriented towards the root of the lower limb and the anterior part of the leg.

An extremity of both peripheral openings 2.5 of the tibial arms 2.1, 2.2 is situated on the "b1" axis, perpendicular to the "a1" axis. The two peripheral openings 2.5 extend towards the anterior part of the knee, in perfect symmetry to the knee itself. Similarly, the two peripheral openings 2.6 of the tibial arms 2.1, 2.2 extend towards the anterior part of the knee, in perfect symmetry to the knee itself. Similarly, the pins 2.7, 2.8 of the lateral tibial arm 2.1 are perfectly symmetrical to the pins 2.7, 2.8 of the medial tibial arm 2.2.

Similarly, the openings 1.9, 1.10 and the pins 1.6, 1.7, 1.8 of the lateral femoral arm 1.1 are perfectly symmetrical to the openings 1.9, 1.10 and to the pins 1.6, 1.7, 1.8 of the medial femoral arm 1.2.

The pins 1.5, 1.6, 1.7, 1.8, present on the medial femoral arm 1.2, are oriented towards the rounded proximal end of the medial tibial arm 2.2, whereas the pins 2.7, 2.8, present on the medial tibial arm 2.2 are oriented towards the rounded distal end of the medial femoral arm 1.2.

The central pin 1.5 of the medial femoral arm 1.2 is engaged in the central opening 2.4 of the medial tibial arm 2.2; the pin 1.6 of the medial femoral arm 1.2 is engaged in the first peripheral opening 2.5 of the medial tibial arm 2.2; the distal pin 1.8 of the medial femoral arm 1.2 is engaged in the second peripheral opening 2.6 of the medial tibial arm 2.2; the distal pin 2.7 of the medial tibial arm 2.2 is engaged in the opening 1.9 of the medial femoral arm 1.2; the proximal pin 2.8 of the medial tibial arm 2.2 is engaged in the opening 1.10 of the medial femoral arm 1.2.

The peripheral edge of the end of the medial tibial arm 2.2, which extends in the quadrants oriented towards the posterior part of the leg, has a particular shape: the initial section of the peripheral edge 2.9 - extending in the posterior quadrant oriented towards the root of the limb - describes a circumference arc. The successive section of the peripheral edge 2.10 - extending in the posterior quadrant oriented towards the foot - is shaped as a spiral that tends to get away from the centre of the plate. More specifically, the points which make up the longitudinal axis of such spiral are situated at an increasingly greater distance from the centre of the rounded proximal end of the medial tibial arm 2.2 as they get near the second extremity. The peripheral surface of the proximal pin 1.7 of the medial femoral arm 1.2 remains in constant contact with the peripheral edge 2.9, 2.10 of the medial tibial arm 2.2 during the roto-traslatory movement of the latter vis-à-vis the medial femoral arm 1.2.

The pins 1.5, 1.6, 1.7, 1.8 and the openings 1.9, 1.10 present on the rounded distal end of the medial femoral arm 1.2, the openings 2.4, 2.5, 2.6 and the pins 2.7, 2.8 present on the rounded distal end of the medial tibial arm 2.2 make up the medial hinge of the device. Similarly, the pins 1.5, 1.6, 1.7, 1.8 and the openings 1.9, 1.10 present on the rounded distal end of the lateral femoral arm 1.1, the openings 2.4, 2.5, 2.6 and the pins 2.7, 2.8 present on the rounded distal end of the lateral tibial arm 2.1 make up the lateral hinge of the device.

The longitudinal axis of the central pin 1.5 of the lateral hinge and the longitudinal axis of the central pin 1.5 of the medial hinge are coaxial. Such axis coincides with the "c" axis which passes through the femoral condyles and around which the rotation of the knee takes place.

The pins 1.5, 1.6, 1.7, 1.8 are each provided with a threaded hole along their longitudinal axis.

The external part of the hinge has a plate 4 with holes that are coaxial with the inner longitudinal holes of the pins 1.5, 1.6, 1.7, 1.8 of the medial femoral arm 1.2. The holes of the plate 4 accommodate the screws that engage the threaded holes of the pins 1.5, 1.6, 1.7, 1.8, thus securing the plate 4 to the medial femoral arm 1.2 and preventing disjunction of the medial tibial arm 2.2 from the medial femoral arm 1.2. The height of the pins 1.5, 1.6, 1.7, 1.8 is greater than the thickness of the medial tibial arm 2.2, which can therefore move freely between the plate 4 and the medial femoral arm 1.2.

Considering that the pins 2.7, 2.8 of the tibial arms 2.1, 2.2 and the pins 1.6, 1.7, 1.8 of the femoral arms 1.1, 1.2 are perfectly symmetrical, the plates 4 of the two hinges have perfectly symmetrical holes too.

As regards the movement in detail, the starting point is the alignment between the medial femoral arm 1.2 and the tibial arm 2.2. Under this condition, the "a" axis of symmetry of the medial femoral arm 1.2 is aligned with the "a1" axis of symmetry of the tibial arm 2.2.

During the first phase of flexion of the leg on the knee, from 0° to 30°:
- the central pin 1.5 of the medial femoral arm 1.2 rotates in the proximal extremity of the central opening 2.4 of the tibial arm 2.2 by the hole placed at the centre of the rounded proximal end of the medial tibial arm 2.2;
- the pin 1.6 of the medial femoral arm 1.2 slides in the circular part of the opening 2.5 of the medial tibial arm 2.2;
- the proximal pin 1.7 of the medial femoral arm 1.2 slides on the circular peripheral edge 2.9 of the medial tibial arm 2.2;
- the distal pin 1.8 of the medial femoral arm 1.2 slides in the circular part of the opening 2.6 of the medial tibial arm 2.2;
- the distal pin 2.7 of the medial tibial arm 2.2 slides in the circular part of the opening 1.9 of the medial femoral arm 1.2;
- the proximal pin 2.8 of the medial tibial arm 2.2 slides in the circular part of the peripheral opening 1.10 of the medial femoral arm 1.2.

In this phase of movement, the hinge performs a rotation movement similar to that of the knee during the first 30 degrees of flexion, which is generally the phase when the foot is fully on the ground.

The subject's bodyweight, which in this phase rests totally on the lower limb, is transmitted, by means of the lateral 1.1 and medial 1.2 femoral arms (secured to the thigh), to the lateral 2.1 and medial 2.2 tibial arms (secured to the leg). In particular, the bodyweight bears on the pins 1.7, 1.8 placed at the distal end of the femoral arms 1.1, 1.2 and on the pins 2.7, 2.8 placed at the proximal end of the tibial arms 2.1, 2.2.

As already pointed out, the knee joint has only two contact points: one between the femoral condyle and the tibial tray in the medial compartment (point A) and the other between the femoral condyle and the tibial tray in the lateral compartment (point B).

Thanks to the device in question, the bodyweight is distributed not only to the physiological contact points A and B, but also to the four pins of the medial hinge and to the four pins of the lateral hinge, placed on both sides of the knee itself. The four pins of each hinge transmit the load at their points of contact with the respective opening 2.6, 1.9, 1.10 and the edge 2.9.

Throughout the various phases of the movement, the specific position of each individual point of contact between pin and opening ensures the suppression of any rotation determined by the distance between the pin and the centre of the hinge.

In case of poor stability, the knee does not usually stick to a circular trajectory, thus modifying the relations between the articulation surfaces, and in particular, the vertical distance. Thanks to the device in question, a circular trajectory is preserved over the first 30 degrees, thereby avoiding a pathological overload of the knee, produced by the subject's bodyweight.

In the second phase of flexion of the leg on the thigh, from 30° to 135°:
- the central pin 1.5 of the medial femoral arm 1.2 slides along the central opening 2.4 of the medial tibial arm 2.2 and reaches its distal extremity;
- the pin 1.6 of the medial femoral arm 1.2 slides in the spiral section of the opening 2.5 of the medial tibial arm 2.2;
- the proximal pin 1.7 of the medial femoral arm 1.2 slides on the spiral peripheral edge 2.10 of the medial tibial arm 2.2;
- the distal pin 1.8 of the medial femoral arm 1.2 slides in the spiral section of the opening 2.6 of the medial tibial arm 2.2;
- the distal pin 2.7 of the medial tibial arm 2.2 slides in the spiral section of the opening 1.9 of the medial femoral arm 1.2;
- the proximal pin 2.8 of the medial tibial arm 2.2 slides in the spiral section of the peripheral opening 1.10 of the medial femoral arm 1.2.

In this phase of movement, the hinge performs a rotatory motion associated with an increasingly progressive sliding, similarly to what happens between the articulation surfaces, which - in a healthy knee - are kept in their proper relations by the cruciate ligaments. There is generally no knee overload in this phase of the movement.

In case of poor stability, however, the knee does not usually stick to a roto-traslatory trajectory, thus modifying the relations between the articulation surfaces. Particularly important to this end is therefore the guiding function imposed to the joint by the two hinges of the device through the pins 1.7, 1.8 placed at the distal end of the femoral arms 1.1, 1.2 and the pins 2.7, 2.8 placed at the proximal end of the tibial arms 2.1, 2.2.

In case of deficient anterior cruciate ligament, leading to the anterior sliding of the tibia (or posterior gliding of the femoral condyle) during flexo-extension, the pins 1.7, 2.7 can be removed, thereby generating a force component of the anterior pins 1.8, 2.8 which - by sliding in their respective openings 2.6, 1.10 - tend to break down the load bearing on the joint into a force component directed towards the centre of the hinge, thus offsetting the anterior sliding of the tibial trays. In this case the inner surface of the femoral plate 1.3 tracks the posterior part of the leg.

In case of deficient posterior cruciate ligament, leading to the posterior sliding of the tibia (or anterior gliding of the femoral condyle) during flexo-extension, the anterior pins 1.8, 2.8 can be removed, thereby generating a force component of the posterior pins 1.7, 2.7 which - by sliding in the opening 1.9 and along the edge 2.9, 2.10 - tend to break down the load bearing on the joint into a force component directed towards the centre of the hinge, thus offsetting the posterior sliding of the tibial trays.

In a second embodiment of the present invention, the medial femoral arm 1.2 is slightly longer than the lateral femoral arm 1.1.

The tibial support 2 consists of a lateral tibial arm 2.1 and a medial tibial arm 2.2, which are parallel to one another and to the longitudinal axis of the leg. They are joined together, however, by means of an arch-shaped tibial plate 2.11 made of harmonic steel. The tibial plate 2.11, placed centrally with respect to the tibial support 2, tracks the anterior part of the leg. The symmetry axis of the tibial plate 2.11 is parallel to the symmetry axis of the femoral plate 1.3.

The medial tibial arm 2.2 consists of two parts: a fixed medial tibial arm 2.12 secured to the tibial plate 2.11 and a free-moving medial tibial arm 2.13 connected to the fixed one by means of a second medial hinge.

The free-moving medial tibial arm 2.13 consists of a rectilinear plate extended between the medial femoral arm 1.2 and the fixed medial tibial arm 2.12. The medial femoral arm 1.2 of the femoral support 1 is connected to the proximal end of the free-moving medial tibial arm 2.13 by means of the medial hinge described in the first embodiment of the present invention.

The end of the free-moving medial tibial arm 2.13 connected to the first medial hinge has the shape of a plate and accommodates the three afore-mentioned openings 2.4, 2.5, 2.6 as well as the two pins 2.7, 2.8. The pins 1.5, 1.6, 1.7, 1.8 and the openings 1.9, 1.10 present on the rounded distal end of the medial femoral arm 1.2, the openings 2.4, 2.5, 2.6 and the pins 2.7, 2.8 present on the rounded proximal end of the free-moving medial tibial arm 2.13 make up the medial hinge of the device. In this case too, the peripheral edge 2.9, 2.10 of the end of the free-moving medial tibial arm 2.13, which extends in the quadrants oriented towards the posterior part of the leg, has a particular shape. The peripheral surface of the proximal pin 1.7 of the medial femoral arm 1.2 remains in constant contact with the peripheral edge 2.9, 2.10 of the free-moving medial tibial arm 2.13 during the roto-traslatory movement of the latter with respect to the medial femoral arm 1.2.

The distal end of the free-moving medial tibial arm 2.13 is rounded and has a hole 2.14 on the symmetry axis of the free-moving medial tibial arm 2.13 itself, which is engaged by a pin 2.15 situated at the intersection between the symmetry axis of the tibial plate 2.11 and the symmetry axis of the fixed medial tibial arm 2.12.

The plate present on the proximal end of the lateral tibial arm 2.1 accommodates the three afore-mentioned openings 2.4, 2.5 e 2.6. The two pins 2.7, 2.8 are also secured to the plate itself. The pins 1.5, 1.6, 1.7, 1.8 and the openings 1.9, 1.10 present on the rounded distal end of the lateral femoral arm 1.1, the openings 2.4, 2.5, 2.6 and the pins 2.7, 2.8 present on the rounded proximal end of the lateral tibial arm 2.1 make up the lateral hinge of the device.

There are, however, some differences between the two peripheral openings 2.5 situated respectively on the lateral tibial arm 2.1 of the tibial support 2 and on the free-moving medial tibial arm 2.13. The circular part of the peripheral opening 2.5 situated on the free-moving medial tibial arm 2.13 has a width of about 15°-20°, while the circular part of the opening 2.5 situated on the lateral tibial arm 2.1 has a width of about 25°-30°. The spiral-shaped part of the peripheral opening 2.5 of the free-moving medial tibial arm 2.13 extends for about 115°-120°, whereas that of the peripheral opening 2.5 situated on the lateral tibial arm 2.1 extends for about 105°-110°. Furthermore, the spiral-shaped part of the peripheral opening 2.5 of the lateral tibial arm 2.1 returns towards the centre of the plate to a greater extent than the spiral-shaped part of the peripheral opening 2.5 of the free-moving medial tibial arm 2.13. The exact extent of this return - to be arranged at the time of construction of the peripheral opening 2.5 of the free-moving medial tibial arm 2.13 - can be established as a result of a radiological study or anthropometric evaluation.

Similarly, the opening 2.6 situated on the free-moving medial tibial arm 2.13 presents a different shape from that of the lateral tibial arm 2.1. The circular part of the peripheral opening 2.6 situated on the free-moving medial tibial arm 2.13 has a width of about 15°-20°, whereas the circular part of the opening 2.6 situated on the lateral tibial arm 2.1 has a width of about 25°-30°. The spiral-shaped part of the peripheral opening 2.6 of the free-moving medial tibial arm 2.13 extends for about 115°-120°, whereas that of the peripheral opening 2.6 situated on the lateral tibial arm 2.1 extends for about 105°-110°. Moreover, the spiral-shaped part of the peripheral opening 2.6 of the lateral tibial arm 2.1 returns towards the centre of the plate to a greater extent than the spiral-shaped part of the peripheral opening 2.6 of the free-moving medial tibial arm 2.13. The opening 2.4 of the lateral tibial arm 2.1 is more elongated than the opening of the free-moving medial tibial arm 2.13.

The central opening 2.4 of the plate of the free-moving medial tibial arm 2.13 is engaged by the central pin 1.5 of the medial femoral arm 1.2. The peripheral opening 2.5 of the plate of the free-moving medial tibial arm 2.13 is engaged by the peripheral pin 1.6 of the medial femoral arm 1.2. The peripheral opening 2.6 of the plate of the free-moving medial tibial arm 2.13 is engaged by the peripheral pin 1.8 of the medial femoral arm 1.2.

The openings 1.9 and 1.10 placed on the lateral femoral arm 1.1 are different from the openings 1.9 and 1.10 placed on the medial femoral arm 1.2. The circular part of the opening 1.9 situated on the lateral femoral arm 1.1 has a width of about 25°-30°, whereas the circular part of the opening 1.9 situated on the medial femoral arm 1.2 has a width of about 15°-20°. The spiral-shaped part of the opening 1.9 situated on the lateral femoral arm 1.1 extends for about 105°-110°, whereas the spiral-shaped part of the opening 1.9 situated on the medial femoral arm 1.2 extends for about 115°-120°.

The spiral-shaped part of the opening 1.9 situated on the medial femoral arm 1.2 returns to a lesser extent towards the centre of the plate with respect to the opening 1.9 situated on the lateral femoral arm 1.1. The spiral-shaped part of the opening 1.10 situated on the medial femoral arm 1.2 returns to a greater extent towards the centre of the plate with respect to the opening 1.10 situated on the lateral femoral arm 1.1. The arc-shaped peripheral edge 2.9 present on the end of the free-moving medial tibial arm 2.13 has a width of about 15-20°, whereas the one present on the lateral tibial arm 2.1 has a width of about 25-30°. The spiral-shaped peripheral edge 2.10 present on the end of the free-moving medial tibial arm 2.13 has a width of 115°-120°, whereas the one present on the lateral tibial arm 2.1 has a width of 105°-110°. The spiral-shaped part of the peripheral edge 2.10 situated on the free-moving medial tibial arm 2.13 returns to a greater extent towards the centre of the plate itself with respect to the spiral-shaped part of the peripheral edge 2.10 situated on the lateral tibial arm 2.1.

An arc-shaped opening 2.16, with rounded extremities, is placed on the symmetry axis of the free-moving medial tibial arm 2.13, in a proximal position vis-à-vis the hole 2.14. The centre of an extremity of the opening 2.16 is placed on the symmetry axis of the free-moving medial tibial arm 2.13. The opening 2.16 extends in anterior direction.

Such opening 2.16 is engaged by a second pin 2.17, which is secured to the fixed medial tibial arm 2.12 and is placed proximally with respect to the pin 2.15 on the symmetry axis of the fixed medial tibial arm 2.12 itself. The pins 2.15, 2.17 have each a threaded hole along their longitudinal axis.

The two due pins 2.15, 2.17 secured to the fixed medial tibial arm 2.12, the hole 2.14 and the arc-shaped opening 2.16 of the free-moving medial tibial arm 2.13 make up the second medial hinge.

The second medial hinge too presents externally a plate 5 with holes, which are coaxial with the inner longitudinal holes of the pins 2.15, 2.17 of the fixed medial tibial arm 2.12. The holes of the plate 5 accommodate the screws which then engage the threaded holes of the pins 2.15, 2.17, securing the plate 5 to the fixed medial tibial arm 2.12 and avoiding the disjunction of the free-moving medial tibial arm 2.13 from the fixed medial tibial arm 2.12. The height of the pins 2.15, 2.17 is greater than the thickness of the free-moving medial tibial arm 2.13, which can therefore move freely between the plate 5 and the fixed medial tibial arm 2.12.

The difference of shape between the openings 2.4, 2.5, 2.6 situated on the free-moving medial tibial arm 2.13 and those situated on the lateral tibial arm 2.1 determines a difference of trajectory between the free-moving medial tibial arm 2.13 and the lateral tibial arm 2.1. The lateral tibial arm 2.1 imposes its trajectory to the fixed medial tibial arm 2.12 to which it is connected by means of the tibial plate 2.11.

During the flexion from 0 to 15-20 degrees, the symmetry axis of the free-moving medial tibial arm 2.13 is coaxial to the symmetry axis of the fixed medial tibial arm 2.12. Both are parallel to the symmetry axis of the lateral tibial arm 2.1.

From 15-20° to 135° of flexion, the symmetry axis of free-moving medial tibial arm 2.13 changes its angle with respect to that of the symmetry axis of the fixed medial tibial arm 2.12. The free-moving medial tibial arm 2.13 starts a roto-traslatory trajectory, imposed by its openings 2.5, 2.6, while the fixed medial tibial arm 2.12 remains on a circular trajectory as the openings 2.5, 2.6 of the lateral tibial arm 2.1 are arc-shaped up to 30 degrees.

The fulcrum of such change of angle between the symmetry axes lies in the pin 2.15 of the fixed medial tibial arm 2.12, which rotates within the hole 2.14 of the free-moving medial tibial arm 2.13 in which it is engaged, whereas the pin 2.17 of the fixed medial tibial arm 2.12 slides in the opening 2.16 of the free-moving medial tibial arm 2.13.

Similarly to what happens in the human knee, the device in question can track the automatic longitudinal rotation of the tibia.

This physiological motion makes the leg and the respective foot rotate externally, thus modifying also the position of the longitudinal axes of the leg itself, which remain always parallel to one another.

The same parallel pattern between the lateral tibial arm 2.1 and the fixed medial tibial arm 2.12 is preserved by the device, which, however, can rotate during flexo-extension by an extent equal to the cosine of the angle formed between the symmetry axis del free-moving medial tibial arm 2.13 and the symmetry axis of the fixed medial tibial arm 2.12, multiplied by the length comprised between the intersection of the "a1", "b1" axes lying on the free-moving medial tibial arm 2.13 and the centre of the hole 2.14, which is engaged by the pin 2.15, situated on the fixed medial tibial arm 2.12, in such a way that the symmetry axis of the fixed medial tibial arm 2.12 and the symmetry axis of the lateral tibial arm 2.1 are always perfectly superimposed to the respective longitudinal axes of the leg, to which they are secured.

The angle of longitudinal rotation can be modified by changing the distance between the intersection of the "a1", "b1" axes lying on the free-moving medial tibial arm 2.13 and the centre of the pin 2.15 of the fixed medial tibial arm 2.12, which must be equal to the distance between the intersection of the "a1", "b1" axes lying on the lateral tibial arm 2.1 and the point of intersection between the symmetry axis of the tibial plate 2.11 and the symmetry axis of the lateral tibial arm 2.1.

In order to improve the adjustment of the device, the distal end of the free-moving medial tibial arm 2.13 accommodate three holes 2.18 along its symmetry axis and three arc-shaped openings 2.19. The position of the free-moving medial tibial arm 2.13 can thus be adjusted with respect to the tibial plate 2.11 by changing the holes 2.18 and the openings 2.19 which are engaged by the pins 2.15, 2.17 of the fixed medial tibial arm 2.12 .

In order to further increase the adjustment of the device in question, the lateral tibial arm 2.1 too consists of a proximal lateral tibial arm 2.20 and a distal lateral tibial arm 2.21. The proximal lateral tibial arm 2.20 has an elongated shape and the distal lateral tibial arm 2.21 is secured to the tibial plate 2.11. Three equidistant holes 2.22 are situated on the prolongation of the proximal lateral tibial arm 2.20 and along its symmetry axis. The distal lateral tibial arm 2.21 accommodates two threaded holes 2.23, coaxial to at least two holes 2.22 present on the proximal lateral tibial arm 2.20. Two screws pass through the holes 2.22 of the proximal lateral tibial arm 2.20 and engage the two threaded holes 2.23 of the distal lateral tibial arm 2.21. The position of the distal lateral arm 2.21 can thus be adjusted with respect to the tibial plate 2.11 by allowing the screws to engage at each time different holes 2.23 of the distal lateral tibial arm 2.21.

## Claims

1. Ergonomic knee device aimed at breaking down the vertical loads, comprising of a femoral support (1) and a tibial support (2); said femoral support (1) including a lateral femoral arm (1.1) and a medial femoral arm (1.2), which are parallel to one another and to the longitudinal axis of the thigh and are joined together by length-adjustable fasteners that secure the femoral support (1) to the thigh; said tibial support (2) including a lateral tibial arm (2.1) and at least one medial tibial arm (2.2, 2.12, 2.13), which are parallel to one another and to the longitudinal axis of the leg;, said femoral arms (1.1, 1.2) and tibial arms (2.2, 2.12) being secured to the thigh and the leg respectively by straps (3) which have a nylon Velcro® face, a plate (1.3, 2.11); said plate (1.3), made of rigid or semirigid material and arc-shaped, tracking with its inner surface the anterior part of the thigh; distal ends of the femoral arms (1.1, 1.2) and proximal ends of the tibial arms (2.1, 2.2, 2.13) being joined together by at least two hinges, one placed laterally and the other medially with respect to the knee; the distal ends of each femoral arm (1.1, 1.2) and the proximal ends of each tibial arm (2.1, 2.2, 2.13), which make up the hinges being plate-shaped and rounded; said device is **characterized by** the fact that said straps (3) pass through linear openings (1.4, 2.3) made on the femoral arms (1.1, 1.2) and tibial arms (2.1, 2.2), the rounded distal end of each femoral arm (1.1, 1.2) accommodating four pins (1.5, 1.6, 1.7, 1.8) and two openings (1.9, 1.10); a first pin (1.5) being placed on the distal end of each femoral arm (1.1, 1.2) in a central position along an "a" axis which coincides with the longitudinal symmetry axis of the femoral arm (1.1, 1.2) itself; a second pin (1.6) being placed on the distal end of each femoral arm (1.1, 1.2) in a peripheral position and at a distance "I" with respect to the first central pin (1.5), along a "b" axis, perpendicular to the "a" axis; the "a" and "b" axes dividing the distal end of the lateral femoral arm (1.1) and of the medial femoral arm (1.2) into four quadrants: a first quadrant oriented towards the foot and the posterior part of the leg, a second quadrant oriented towards the foot and the anterior part of the leg, a third quadrant oriented towards the root of the lower limb and the posterior part of the leg and a fourth quadrant oriented towards the root of the lower limb and the anterior part of the leg; a third pin (1.7) being situated proximally with respect to the first and second pins (1.5, 1.6) and in the third quadrant oriented towards the root of the lower limb and the posterior part of the leg; said third proximal pin (1.7) being placed at a distance "r" with respect to the first central pin (1.5); a "d" axis, which passes through a centre of the third proximal pin (1.7) and a centre of the first central pin (1.5), deviating by a few degrees from the "a" axis, a peripheral edge of the third proximal pin (1.7) being tangential to the "a" axis; a fourth pin (1.8) being situated in the second quadrant oriented towards the foot and the anterior part of the leg, on the prolongation of said "d" axis at a distance "r" with respect to the first central pin (1.5); a peripheral edge of the fourth distal pin (1.8) being tangential to the "a" axis and is diametrically opposed to the third proximal pin (1.7) with respect to the first central pin (1.5); said first opening (1.9) being situated near a peripheral edge of the rounded distal end of each femoral arm (1.1, 1.2) and extending into the first and third quadrants oriented towards the posterior part of the leg for 130°-140 degrees; a centre of a first semicircular extremity of the first opening (1.9) being placed at a distance "r" from the first central pin (1.5) along a "d1" axis, which is symmetrical to the "d" axis vis-à-vis the "a" axis; an edge of the first semicircular extremity of said first opening (1.9) being tangential to the "a" axis and to the peripheral edge of the fourth distal pin (1.8); starting from said first extremity over the first 25-30 degrees, said first opening (1.9) having the shape of an arc of circumference; from 25-30 degrees up to 130-140 degrees, said first opening (1.9) having the shape of a spiral which nears a centre of the rounded distal end of the lateral femoral arm (1.1) and of the medial femoral arm (1.2); said second opening (1.10) being diametrically opposed to said first opening (1.9) with respect to the "a" axis and placed in the second and fourth quadrants oriented towards the anterior part of the leg; a centre of a first extremity of said second opening (1.10) being placed at a distance "r" from the first central pin (1.5) along said "d1" axis; an edge of the first semicircular extremity of said second opening (1.10) being tangential to the "a" axis and to the peripheral edge of the third proximal pin (1.7); starting from said first semicircular extremity for the first 25-30 degrees, said second opening (1.10) having the shape of an arc of circumference; from 25-30 degrees up to 130-140 degrees, said second opening (1.10) having the shape of a spiral that, unlike the first opening (1.9), gets away from the centre of the rounded distal end of the femoral arm (1.1 1.2); the plate present on the proximal end of each tibial arm (2.1, 2.2, 2.13) accommodating three openings (2.4, 2.5, 2.6) and two pins (2.7, 2.8); the first tibial opening (2.4) having a rectangular shape with rounded extremities and being obtained by making a hole at a centre of the plate of the proximal end of the medial tibial arm (2.1, 2.2, 2.13) and proceeding towards the foot along a "a1" axis of longitudinal symmetry of the tibial arm (2.1, 2.2, 2.13); a second opening (2.5) extending for about 130-140 degrees and being placed peripherally on the proximal end of each tibial arm (2.1, 2.2, 2.13); a first extremity of said second peripheral opening (2.5) being situated on a "b1" axis, perpendicular to the "a1" axis, at a distance "l" from the centre of the central hole, which originates the first opening (2.4); another extremity of the second tibial opening (2.5) being placed at 130-140 degrees with respect to the "b1" axis; starting from said "b1" axis, for the first 25-30 degrees, said second tibial opening (2.5) having the shape of an arc of circumference with a centre that coincides with that of the hole generating the first central tibial opening (2.4) and with a radius that is equal to "l"; from 25-30 degrees to the remaining 105-110 degrees said second tibial opening (2.5) having the shape of a spiral that returns towards the centre of the proximal end of each tibial arm (2.1, 2.2, 2.13); a longitudinal axis of said spiral being the result of the sequence of points covered by an extremity of a segment having a length "l" whose second extremity travelling along a longitudinal axis of the first central opening (2.4) from a centre of each tibial arm (2.1, 2.2, 2.13) to the periphery; the "a1" axis and the "b1" axis dividing the proximal end of each tibial arm (2.1, 2.2, 2.13) into four quadrants: a first quadrant being oriented towards the foot and the posterior part of the leg, a second quadrant being oriented towards the foot and the anterior part of the leg, a third quadrant being oriented towards the root of the lower limb and the posterior part of the leg and a fourth quadrant being oriented towards the root of the lower limb and the anterior part of the leg; said central tibial opening (2.4) being placed in the first and second quadrants oriented towards the foot, said second tibial opening (2.5) placed in the third and fourth quadrants oriented towards the root of the lower limb; a centre of a first semicircular extremity of a third tibial opening (2.6) being situated at a distance "r" from an intersection of the "a1" and "b1" axes in the second quadrant oriented towards the foot and the anterior part of the leg, and deviating by a few degrees from the "a1" axis; an edge of an extremity of said third tibial opening (2.6) being tangential to the "a1" axis; said third tibial opening (2.6) extending for about 130-140 degrees and for the first 25-30 degrees having the shape of an arc of circumference, from 25-30 degrees up to 130-140 degrees said third tibial opening (2.6) having the shape of a spiral which nears the centre of the rounded proximal end of each tibial arm (2.1, 2.2, 2.13); a first tibial pin (2.7) being placed distally with respect to the first tibial central opening (2.4) and at a distance "r" from the intersection of "a1" and "b1" axes in the first quadrant oriented towards the foot and the posterior part of the leg; a "d2" axis, which passes through the centre of the first tibial pin (2.7) and the intersection of the "a1" and "b1" axes, deviating by a few degrees from the "a1" axis; a peripheral edge of the first tibial pin (2.7) being tangential to the "a1" axis and to the first extremity of the third tibial opening (2.6); said "d2" axis extending towards a proximal peripheral edge of the medial tibial arm (2.2); a second tibial pin (2.8) being placed on said "d2" axis at a distance "r" with respect to the intersection of the "a1" and "b1" axes; said second tibial pin (2.8) being diametrically opposed to the first tibial pin (2.7) vis-à-vis the "a1" axis, consequently the "d2" axis, which passes through a centre of the second tibial pin (2.8) and the intersection of the "a1" and "b1" axes and deviating by a few degrees from the "a1" axis in the opposite direction; a peripheral edge of the second tibial pin (2.8) being tangential to the "a1" axis; said second tibial pin (2.8) being placed in the fourth quadrant oriented towards the root of the lower limb and the anterior part of the leg; an extremity of both peripheral second openings (2.5) of the tibial arms (2.1, 2.2, 2.13) being situated on the "b1" axis which is perpendicular to the "a1" axis; the two peripheral second openings (2.5) extending towards the anterior part of the knee and being symmetrical to the knee; the two peripheral third openings (2.6) of the tibial arms (2.1, 2.2, 2.13) extending towards the anterior part of the knee and being symmetrical to the knee itself; the first and second pins (2.7, 2.8) of the lateral tibial arm (2.1) being symmetrical to the first and second pins (2.7, 2.8) of the medial tibial arm (2.2, 2.13), the first and second openings (1.9, 1.10) and the second, third and fourth pins (1.6, 1.7, 1.8) of the lateral femoral arm (1.1) being symmetrical to the first and second openings (1.9, 1.10) and the second, third and fourth pins (1.6, 1.7, 1.8) of the medial femoral arm (1.2); said first, second, third and fourth pins (1.5, 1.6, 1.7, 1.8) present on each femoral arm (1.1, 1.2) being oriented towards the rounded proximal end of each tibial arm (2.1, 2.2, 2.13), the first and second pins (2.7, 2.8) present on each tibial arm (2.1, 2.2, 2.13) being oriented towards the rounded distal end of each femoral arm (1.1, 1.2); a first part of a peripheral edge (2.9) of the end of each tibial arm (2.1, 2.2, 2.13), which extends in the quadrant oriented towards the root of the lower limb, having the shape of an arc of circumference; a second part of a peripheral edge (2.10) of the end of each tibial arm (2.1, 2.2, 2.13), which extends in the posterior quadrant oriented towards the foot, having the shape of a spiral which gets away from a centre of the end of each tibial arm (2.1, 2.2, 2.13); a peripheral surface of said third proximal pin (1.7) of each femoral arm (1.1, 1.2) remaining in constant contact with the peripheral edge (2.9, 2.10) of each tibial arm (2.1, 2.2, 2.13) during roto-traslatory movement of each tibial arm (2.1, 2.2, 2.13) with respect to each femoral arm (1.1, 1.2); the first central pin (1.5) of each femoral arm (1.1, 1.2) engaging the first central opening (2.4) of each tibial arm (2.1, 2.2, 2.13); the second pin (1.6) of each femoral arm (1.1, 1.2) engaging the second peripheral opening (2.5) of each tibial arm (2.1, 2.2, 2.13); the fourth distal pin (1.8) of each femoral arm (1.1, 1.2) engaging the third peripheral opening (2.6) of each tibial arm (2.1, 2.2, 2.13); the first distal tibial pin (2.7) of each tibial arm (2.1, 2.2, 2.13) engaging the first opening (1.9) of each femoral arm (1.1, 1.2), the second proximal pin (2.8) of each tibial arm (2.1, 2.2, 2.13) engaging the second opening (1.10) of each femoral arm (1.1, 1.2); the first, second, third and fourth pins (1.5, 1.6, 1.7, 1.8) and the first and second openings (1.9, 1.10) on the rounded distal end of the lateral femoral arm (1.1) and the first, second and third openings (2.4, 2.5, 2.6) and the first and second pins (2.7, 2.8) on the rounded proximal end of the lateral tibial arm (2.1) making up a lateral hinge of the device; the first, second, third and fourth pins (1.5, 1.6, 1.7, 1.8) and the first and second openings (1.9, 1.10) on the rounded distal end of the medial femoral arm (1.2), the first, second and third openings (2.4, 2.5, 2.6) and the first and second pins (2.7, 2.8) on the rounded proximal end of the medial tibial arm (2.2, 2.13) making up a medial hinge of the device; said first, second, third and fourth pins (1.5, 1.6, 1.7, 1.8) of the distal ends of the femoral arms (1.1, 1.2) being secured to a plate (4) placed externally to each tibial arm (2.1, 2.2, 2.13) by means of fasteners; each plate (4) being provided with holes that are coaxial with the first, second, third and fourth pins (1.5, 1.6, 1.7, 1.8) of each femoral arm (1.1, 1.2); said holes of the plate (4) accommodating free ends of the first, second, third and fourth pins (1.5, 1.6, 1.7, 1.8) or the fasteners which engage the first, second, third and fourth pins (1.5, 1.6, 1.7, 1.8), thus securing each plate (4) to each femoral arm (1.1, 1.2) and avoiding disjunction of each plate (4) from the respective tibial arm (2.1, 2.2, 2.13); the height of the first, second, third and fourth pins (1.5, 1.6, 1.7, 1.8) being greater than the thickness of each tibial arm (2.1, 2.2, 2. 13) so that each tibial arm (2.1, 2.2, 2. 13) can move freely between the plate (4) and the femoral arm (1.1, 1.2).

2. Ergonomic knee device, in accordance with claim 1, **characterized by** the lateral tibial arm (2.1) and the medial tibial arm being joined together by an arch-shaped tibial plate (2.11) made of rigid material with a good harmonic response like harmonic steel; said tibial plate (2.11) being placed in central position with respect to the tibial support (2) and tracking the anterior part of the leg; a symmetry axis of the tibial plate (2.11) being parallel to a symmetry axis of the femoral plate (1.3); the medial tibial arm having two parts: a fixed medial tibial arm (2.12) secured to the tibial plate (2.11) and a free-moving medial tibial arm (2.13) secured to the fixed medial tibial arm (2.12) by means of a second medial hinge; the free-moving medial tibial arm (2.13) including a rectilinear plate which extends between the medial femoral arm (1.2) and the fixed medial tibial arm (2.12); the fixed medial femoral arm (1.2) of the femoral support (1) being connected to a proximal end of the free-moving medial tibial arm (2.13) by means of a medial hinge including the first, second, third and fourth pins (1.5, 1.6, 1.7, 1.8) and the first and second openings (1.9, 1.10) on the rounded distal end of the medial femoral arm (1.2), as well as the first, second and third openings (2.4, 2.5, 2.6) and the first and second pins (2.7, 2.8) on the rounded proximal end of the free-moving medial tibial arm (2.13); a distal end of the free-moving medial arm (2.13) being rounded and is provided with a hole (2.14) situated on the symmetry axis of the free-moving medial tibial arm (2.13), which is engaged by a first pin (2.15) placed at the intersection between the symmetry axis of the tibial plate (2.11) and a symmetry axis of the fixed medial tibial arm (2.12); an opening (2.16), which has the shape of a circumference arc and has rounded extremities, being placed on the symmetry axis of the free-moving medial tibial arm (2.13), proximally to the hole (2.14); a centre of an extremity of the opening (2.16) being situated on the symmetry axis of the free-moving medial tibial arm (2.13); the opening (2.16) extending anteriorly to the knee and accommodating a second pin (2.17), secured to the fixed medial tibial arm (2.12) and placed proximally with respect to the first pin (2.15) on the symmetry axis of the fixed medial tibial arm (2.12); the first and second pins (2.15, 2.17) secured to the fixed medial tibial arm (2.12), the hole (2.14) and the arc-shaped opening (2.16) of the free-moving medial tibial arm (2.13) making up a second medial hinge; said first and second pins (2.15, 2.17) of the fixed medial tibial arm (2.12) being secured to an external plate (5) by means of fasteners; the plate (5) being provided with holes coaxial with the first and second pins (2.15, 2.17) secured to the fixed medial tibial arm (2.12); said holes of the plate (5) accommodating free ends of the first and second pins (2.15, 2.17) or the fasteners which engage the first and second pins (2.15, 2.17) securing the plate (5) to the fixed medial tibial arm (2.12) and preventing the disjunction of the free-moving medial tibial arm (2.13) from the fixed medial tibial arm (2.12); the height of the first and second pins (2.15, 2.17) being greater than the thickness of the free-moving medial tibial arm (2.13), which can consequently travel freely between the plate (5), the free-moving medial tibial arm (2.13) and the fixed medial tibial arm (2.12); the medial femoral arm (1.2) being slightly longer than the lateral femoral arm (1.1).

3. Ergonomic knee device, in accordance with the claim 2, **characterized by** the circular part of the second peripheral opening (2.5) situated on the free-moving medial tibial arm (2.13) having a width of about 15°-20°, the circular part of the second opening (2.5) situated on the lateral tibial arm (2.1) having a width of about 25°-30°; the spiral-shaped part of the second peripheral opening (2.5) of the free-moving medial tibial arm (2.13) extending for about 115°-120°, the spiral-shaped part of the second peripheral opening (2.5) situated on the lateral tibial arm (2.1) extending for about 105°-110°; the spiral-shaped part of the peripheral opening (2.5) of the lateral tibial arm (2.1) returning towards a centre of the plate to a greater extent compared to the spiral-shaped part of the second peripheral opening (2.5) of the free-moving medial tibial arm (2.13); the circular part of the third peripheral opening (2.6) situated on the free-moving medial tibial arm (2.13) having a width of about 15°-20°, the circular part of the third opening (2.6) situated on the lateral tibial arm (2.1) having a width of about 25°-30°; the spiral-shaped part of the third peripheral opening (2.6) of the free-moving medial tibial arm (2.13) extending for about 115°-120°, the spiral-shaped part of the third peripheral opening (2.6) situated on the lateral tibial arm (2.1) extending for about 105°-110°; the spiral-shaped part of the third peripheral opening (2.6) of the lateral tibial arm (2.1) returning towards the centre of the plate to a greater extent compared to the spiral-shaped part of the third peripheral opening (2.6) of the free-moving medial tibial arm (2.13); the first opening (2.4) of the lateral tibial arm (2.1) having a more elongated shape than the opening of the free-moving medial tibial arm (2.13); the circular part of the first opening (1.9) situated on the lateral femoral arm (1.1) having a width of about 25°-30°, the circular part of the first opening (1.9) situated on the medial femoral arm (1.2) having a width of about 15°-20°; the spiral-shaped part of the first opening (1.9) situated on the lateral femoral arm (1.1) extending for about 105°-110°, the spiral-shaped part of the first opening (1.9) situated on the medial femoral arm (1.2) extending for about 115°-120°; the spiral-shaped part of the first peripheral opening (1.9) of the lateral femoral arm (1.1) returning towards the centre of the plate to a greater extent compared to the spiral-shaped part of the first peripheral opening (1.9) of the medial femoral arm (1.2); the circular part of the second opening (1.10) situated on the lateral femoral arm (1.1) having a width of about 25°-30°, the circular part of the second opening (1.10) situated on the medial femoral arm (1.2) having a width of about 15°-20°; the spiral-shaped part of the second opening (1.10) situated on the lateral femoral arm (1.1) extending for about 105°-110°, the spiral-shaped part of the second opening (1.10) situated on the medial femoral arm (1.2) extending for about 115°-120°; the spiral-shaped part of the second peripheral opening (1.10) of the lateral femoral arm (1.1) returning towards the centre of the plate to a lesser extent compared to the spiral-shaped part of the second peripheral opening (1.10) of the medial femoral arm (1.2); the arc-shaped peripheral first edge (2.9) on the end of the free-moving medial tibial arm (2.13) having a width of 15-20°, the arc-shaped peripheral first edge (2.9) on the lateral tibial arm (2.1) having a width of 25-30°; the spiral-shaped peripheral second edge (2.10) present on the end of the free-moving medial tibial arm (2.13) having a width of 115°-120°, the spiral-shaped peripheral second edge (2.10) on the lateral tibial arm (2.1) having a width of 105°-110°; the spiral-shaped part of the peripheral second edge (2.10) situated on the free-moving medial tibial arm (2.13) returning towards the centre of the plate to a greater extent compared to the spiral-shaped peripheral second edge (2.10) situated on the lateral tibial arm (2.1).

4. Ergonomic knee device, in accordance with the claims 1 and 2, **characterized by** the pins (1.5, 1.6, 1.7, 1.8) of the distal ends of the femoral arms (1.1, 1.2) and the first and second pins (2.15, 2.17) of the fixed medial tibial arm (2.12) being each provided with a threaded hole along longitudinal axis thereof; said holes of the plates (4, 5) accommodating the screws that engage the threaded holes of the pins (1.5, 1.6, 1.7, 1.8, 2.15, 2.17).

5. Ergonomic knee device, in accordance with the claims 1 and 2, **characterized by** the pins (1.5, 1.6, 1.7, 1.8) of the distal ends of the femoral arms (1.1, 1.2) and the first and second pins (2.15, 2.17) of the fixed medial tibial arm (2.12) being each provided with a hole along longitudinal axis thereof; the holes of the plates (4, 5) accommodating the rivets each having a free end that engages the holes of the pins (1.5, 1.6, 1.7, 1.8, 2.15, 2.17).

6. Ergonomic knee device, in accordance with the claims 1 and 2, **characterized by** the pins (1.5, 1.6, 1.7, 1.8) of the distal ends of the femoral arms (1.1, 1.2) and the first and second pins (2.15, 2.17) of the fixed medial tibial arm (2.12) being each provided with an external thread on a free end thereof; each one of said threads being engaged by a nut.

7. Ergonomic knee device, in accordance with the claim 1, **characterized by** the pins (1.5, 1.6, 1.7, 1.8, 2.15, 2.17) of the femoral arms (1.1, 1.2) and of the medial tibial arms (2.1, 2.2, 2.13) being able to be removed in the presence of specific pathologies.

8. Ergonomic knee device, in accordance with the claim 2, **characterized by** the tibial plate (2.11) being able to be connected to each tibial arm (2.2, 2.3) by means of a fastener.

9. Ergonomic knee device, in accordance with the claim 2, **characterized by** the distal end of the free-moving medial tibial arm (2.13) accommodating multiple holes (2.18) along the symmetry axis thereof and multiple arc-shaped openings (2.19); a centre of a semicircular extremity of the openings (2.19) being placed along the same symmetry axis of the free-moving medial tibial arm (2.13).

10. Ergonomic knee device, in accordance with the claim 2, **characterized by** the lateral tibial arm including a proximal lateral tibial arm (2.20) and a distal lateral tibial arm (2.21); the proximal lateral tibial arm (2.20) having an elongated shape, the distal lateral tibial arm (2.21) being secured to the tibial plate (2.11); at least three equidistant holes (2.22) being placed on the prolongation of the proximal lateral tibial arm (2.20) and along a symmetry axis thereof; at least two threaded holes (2.23) being placed on the distal lateral tibial arm (2.21) and being coaxial to at least two holes (2.22) on the proximal lateral tibial arm (2.20); two screws engaging the holes (2.22) of the proximal lateral tibial arm (2.20) and being secured in the two threaded holes (2.23) of the distal lateral tibial arm (2.21).

## Patentansprüche

1. Ergonomische Knie-Vorrichtung zur Behebung von vertikalen Lasten, bestehend aus einem Oberschenkelhalter (1) und aus einem Unterschenkelhalter (2), wobei der Oberschenkelhalter (1) aus einem seitlichen Oberschenkelarm (1.1) und aus einem medialen Oberschenkelarm (1.2) besteht, die untereinander und zu der Längsachse des Oberschenkels parallel sind sowie durch in der Länge einstellbare Mittel verbunden sind, die dazu geeignet sind, den Oberschenkelhalter (1) auf dem Oberschenkel und den genannten Unterschenkelhalter (2) zu befestigen, der aus einem seitlichen Unterschenkelarm (2.1) und mindestens aus einem medialen Unterschenkelarm (2.2, 2.12, 2.13) besteht, die untereinander und zu Längsachse des Beins parallel sind; diese Oberschenkelarme (1.1, 1.2) und Unterschenkelarme (2.2, 2.12) sind auf dem Oberschenkel bzw. auf dem echten Bein mittels Gurten (3) befestigt, die eine Velcro®-Nylon-Seite, die durch in den Oberschenkelarme (1.1, 1.2) und Unterschenkelarme (2.1, 2.2) angebrachte lineare Schlitzen verlaufen, und ein Band (1.3, 2.11) haben; dieses bogenförmige Band (1.3) aus einem harten oder halbharten Material umläuft mit seiner inneren Oberfläche die Vorderseite des Oberschenkels; die distalen Enden der Oberschenkelarme (1.1, 1.2) und die proximalen Enden der Unterschenkelarme (2.1, 2.2, 2.13) werden durch zwei Gelenke, eine seitlich und eine medial zum Knie, bewegt; die distalen Enden jedes Oberschenkelarms (1.1, 1.2) und die proximalen Enden der Unterschenkelarme (2.1, 2.2, 2.13), die die Gelenke bilden, sind als Platten geformt und abgerundet; diese Ausrüstung kennzeichnet sich **dadurch**, dass auf dem distalen abgerundeten Ende jedes Oberschenkelarmes (1.1, 1.2) vier Stifte (1.5, 1.6, 1.7, 1.8) befestigt sind und zwei Öffnungen (1.9, 1.10) realisiert sind; eine erste Stift (1.5) befindet sich zentral auf dem distalen Ende jedes Oberschenkelarmes (1.1., 1.2), entlang einer "a"-Achse, die dem Symmetrie-Längsachse desselben Oberschenkelarmes (1.1, 1.2) entspricht; eine zweite Stift (1.6) befindet sich versetzt auf dem distalen Ende jedes Oberschenkelarmes (1.1., 1.2) und zu einer "l"-Abstand von der Stift (1.5), zentral, entlang einer der "a"-Achse orthogonalen "b"-Achse; die "a"-Achse und die "b"-Achse teilen ideal das distale Ende des Oberschenkelarmes (1.1) seitlich und des Unterschenkelarmes (1.2) medial in vier Quadranten, der erste gegen den Fuß und den hinteren Teil des Beines gerichtet, der zweite gegen den Fuß und den vorderen Teil des Beines gerichtet, der dritte gegen die untere Gliedwurzel und gegen den hinteren Teil des Beines und der vierte gegen die untere Gliedwurzel und gegen den vordere Teil des Beins gerichtet; eine dritte Stift (1.7) befindet sich in proximaler Lage zu den oben beschriebenen Stiften (1.5, 1.6) und im Quadranten, der gegen die untere Gliedwurzel und den unteren Teil des Beines gerichtet ist; diese proximale Stift (1.7) befindet sich auf einer "r"-Abstand zur zentralen Stift (1.5); die "d"-Achse, die durch das Zentrum der proximalen Stift (1.7) und durch der mittleren Stift (1.5) verläuft, weicht von einigen Graden von der "a"-Achse ab und der peripherische Rand der proximalen Stift (1.7) tangiert die selbe "a"-Achse; diese vierte Stift (1.8) befindet auf dem Quadrant, der gegen den Fuß und den vorderen Teil des Beines gerichtet ist, auf der Verlängerung dieser "a"-Achse auf einer "r"-Abstand zur zentralen Stift (1.5); der peripherische Rand der distalen Stift (1.8) tangiert mit der "a"-Achse und befindet sich auf der gegenüberliegenden Seite der proximalen Stift (1.7) zur zentralen Stift (1.5); diese erste Öffnung (1.9) befindet sich neben dem peripherischen Rand der distalen abgerundeten Ende jedes Unterschenkelarmes (1.1, 1.2) und entwickelt sich in den zwei Quadranten, die gegen den unteren Teil des Beins für 130°-140° gerichtet sind; das Zentrum eines ersten halbrunden Endes der Öffnung (1.9) befindet sich auf einer "r"-Abstand von der mittleren Stift (1.5) entlang einer "d1"-Achse, spiegelbildlich zu der "d"-Achse zur "a"-Achse; der Rand des ersten halbrunden Endes der ersten Öffnung (1.9) tangiert mit der "a"-Achse und mit dem peripherischen Rand der distalen Stift (1.8); ausgehend von diesem ersten Ende sind die ersten 25°-30° der ersten Öffnung (1.9) wie ein Kreisbogen geformt; von 25°-30° bis 130°-140° ist die erste Öffnung (1.9) wie eine Spiral geformt, die sich dem Zentrum des distalen gerundeten Endes des seitlichen Oberschenkelarmes (1.1) und des medialen Oberschenkelarmes (1.2) nähert; die zweite Öffnung (1.10) befindet sich genau gegenüber der oben genannten ersten Öffnung (1.9) zur "a"-Achse und in den zwei Quadranten, die gegen den vorderen Teil des Beines gerichtet ist; das Zentrum eines ersten Endes der zweiten Öffnung (1.10) befindet sich auf einer "r"-Abstand von der mittleren Stift (1.5) entlang der genannten "d1 "-Achse; der Rand des ersten halbrunden Endes der genannten zweiten Öffnung (1.10) tangiert mit der "a"-Achse und mit dem peripherischen Rand der proximalen Stift (1.7); ausgehend von diesem ersten halbrunden Ende sind die erste 25°-30° dieser zweiten Öffnung (1.10) wie ein Kreisbogen geformt; diese zweite Öffnung (1.10) ist von 25°-30° bis 130°-140° wie eine Spiral geformt, die, im Vergleich zur ersten Öffnung (1.9) sich von dem Zentrum des distalen gerundeten Endes des Oberschenkelarmes (1.1, 1.2) entfernt; auf der Platte, die sich am proximalen Ende jedes Unterschenkelarmes (2.1, 2.2) befindet, sind drei Öffnungen (2.4, 2.5, 2.6) vorhanden und auf dieser Platte sind zwei Stifte (2.7, 2.8) befestigt; die erste Unterschenkelöffnung (2.4) ist rechteckig, mit abgerundeten Enden und wird durch Bohren des Zentrums der Platte im proximalen Ende des medialen Unterschenkelarmes (2.1, 2.2, 2.13) erstellt und weiter Richtung Fuß der "a1"-Achse entlang, länglich symmetrisch zum Unterschenkelarm (2.1, 2.2, 2.13); die zweite Öffnung (2.5) erstreckt sich für ca. 130°-140° und befindet sich am Rand des proximalen Endes jedes Unterschenkelarmes (2.1, 2.2, 2.13); ein erstes Ende dieser zweiten Randöffnung (2.5) befindet sich auf einer "b1 "-Achse, orthoganal zur "a1"-Achse, zu einer "l"-Abstand vom Zentrum des obengenannten mittleren Zentrum, aus dem die erste Öffnung entsteht (2.4); das andere Ende der zweiten Unterschenkelöffnung (2.5) befindet sich bei 130-140° zur "b1 "-Achse; ausgehend von dieser "b1 "-Achse ist diese zweite Unterschenkelöffnung (2.5) für die ersten 25°-30° wie ein Kreisbogen geformt, dessen Zentrum dem Zentrum der Öffnung, aus der der mittlere Unterschenkelarm (2.4) entsteht, entspricht und der ein mit "l" entsprechendes Radius hat; aus den 25°-30° bis zu den restlichen 105-110° ist es wie eine Spirale gestaltet, die gegen das Zentrum des proximalen Endes jedes Unterschenkelarmes (2.1, 2.2, 2.13) einrückt; die Längsachse dieser Spirale entsteht aus der Folge der Punkten des Endes eines "l"-langen Segmentes, dessen zweite Ende sich entlang der Längsachse der zentralen Öffnung (2.4) bewegt, weiter aus dem Zentrum jedes Unterschenkelarmes (2.1, 2.2, 2.13) bis zum Randbereich; die vorgenannte "a1 "-Achse und die vorgenannte "b1 "-Achse teilen ideal das proximale Ende des Unterschenkelarmes (2.1, 2.2, 2.13) in vier Quadranten, der erste gegen den Fuß und den hinteren Teil des Beines gerichtet, der zweite gegen den Fuß und den vorderen Teil des Beines gerichtet, der dritte gegen die untere Gliedwurzel und gegen den hinteren Teil des Beines und der vierte gegen die untere Gliedwurzel und gegen den vordere Teil des Beins gerichtet; die obengenannte zentrale Unterschenkelöffnung (2.4) befindet sich in den beiden Quadranten, die gegen den Fuß gerichtet sind, während die obengenannte Unterschenkelöffnung (2.5) sich in den zwei Quadranten, die gegen den unteren Gliedwurzel gerichtet sind, befindet; das Zentrum eines ersten halbrunden Endes der dritten Öffnung (2.6) befindet sich auf einer "r"-Abstand von der Kreuzung der "a1" u. "b1"-Achsen im Quadranten, der gegen den Fuß und den vorderen Teil des Beines gerichtet ist und von einigen Graden von der "a1 "-Achse abweicht; der Rand des Endes der dritten Unterschenkelöffnung (2.6) tangiert mit der "a1"-Achse; diese dritte Unterschenkelöffnung (2.6) erstreckt sich für ca. 130°-140° und ist ein Kreisbogen für die ersten 25°-30°, während dies von den 25°-30° bis zu den 130°-140° wie eine Spirale ausgebildet ist, die sich dem Zentrum des proximalen abgerundeten Endes jedes Unterschenkelarmes (2.1, 2.2, 2.13) nähert; die erste Unterschenkelstift (2.7) befindet sich distal zu der zentralen Öffnung (2.4) und auf einer "r"-Abstand von der Kreuzung der "a1" u. "b1"-Achsen im Quadranten, der gegen den Fuß und den vorderen Teil des
Beines gerichtet ist; die "d2"-Achse, die durch das Stiftzentrum (2.7) verläuft und die Kreuzung der "a1"- und "b1"-Achse, weicht von einigen Graden von der "a1"-Achse ab; der peripherische Rand der ersten Unterschenkelstift (2.,7) tangiert mit derselben "a1"-Achse und mit dem ersten Ende der dritten Unterschenkelöffnung (2.6); diese "d2"-Achse erstreckt sich gegen den peripherischen proximalen Rand des medialen Unterschenkelarmes (2.2). Auf dieser Achse, auf einer "r"-Abstand zu der Kreuzung der "a1"- u. "b1"-Achsen befindet sich eine zweite Unterschenkelstift (2.8); diese zweite Unterschenkelstift (2.8) befindet sich genau gegenüber der ersten Unterschenkelstift (2.7) zur "a1"-Achse und somit weicht die "d2"-Achse, die durch das Zentrum der Stift 2.8 verläuft, ab und die Kreuzung der "a1"- u. "b1"-Achsen weicht von einigen Graden von der "a1"-Achse, jedoch in entgegengesetzter Richtung; auch der peripherischen Rand der zweiten Stift (2.8) tangiert mit der "a1"-Achse; diese zweite proximale Unterschenkelstift (2.8) befindet sich im Quadranten, der gegen die untere Gliedwurzel und den unteren Teil des Beines gerichtet ist; ein Ende beider Randöffnungen (2.5) der Unterschenkelarme (2.1, 2.2, 2.13) befindet sich auf der "b1 "-Achse, die orthoganal zur "a1"-Achse ist; beide Randöffnungen (2.5) erstrecken sich gegen den vorderen Teil des Knies und spiegelbildlich zu ihm; auch beide Randöffnungen (2.6) der Unterschenkelarme (2.1, 2.2, 2.13) erstrecken sich gegen den vorderen Teil des Knies und spiegelbildlich zu ihm; analog sind die Stifte (2.7, 2.8) des seitlichen Unterschenkelarmes (2.1) spiegelbildlich zu den Stiften (2.7, 2.8) des medialen Unterschenkelarmes (2.2, 2.13) positioniert und die Öffnungen (1.9, 1.10) sowie die Stifte (1.6, 1.7, 1.8) des seitlichen Oberschenkelarmes (1.1) sind spiegelbildlich zu den Öffnungen (1.9, 1.10) und zu den Stiften (1.6, 1.7, 1.8) des medialen Oberschenkelarmes (1.2) positioniert; diese Stifte (1.5, 1.6, 1.7, 1.8), die sich auf jedem Oberschenkelarm (1.1, 1.2) befinden, sind gegen das proximale abgerundete Ende jedes Unterschenkelarmes (2.1, 2.2, 2.13) gerichtet, während die Stifte (2.7, 2.8), die sich auf jedem Unterschenkelarm (2.1, 2.2, 2.13) befinden, gegen das distale abgerundete Ende jedes Oberschenkelarmes (1.1, 1.2) gerichtet sind; ein erster Teil des peripherischen Randes (2.9) am Ende jedes Unterschenkelarmes (2.1, 2.2, 2.13), der sich im hinteren gegen den Gliedwurzel gerichteten Beinquadranten entwickelt, ist wie ein Kreisbogen gestaltet; in einem zweien Teil, der sich im hinteren gegen den Fuß gerichteten Fuß befindet, ist der peripherischer Rand (2.10) spiralförmig gestaltet, wobei diese Spirale dazu neigt, sich von dem Zentrum des Endes jedes Unterschenkelarmes zu entfernen (2.1, 2.2, 2.13); die Randoberfläche dieser proximalen Stift (1.7) jedes Oberschenkelarms (1.1, 1.2) berührt immer den peripherischen Rand (2.9, 2.10) jedes Unterschenkelarms (2.1, 2.2, 2.13) während dessen DrehBewegung im Vergleich zum jeden Oberschenkelarm (1.1, 1.2); die zentrale Stift (1.5) jedes Oberschenkelarmes (1.1, 1.2) fügt sich in die zentrale Öffnung (2.4) jedes Unterschenkelarmes (2.1, 2.2, 2.13) ein, die zweite Stift (1.6) jedes Oberschenkelarmes (1.1, 1.2) fügt sich in die erste Randöffnung (2.5) jedes Unterschenkelarmes (2.1, 2.2, 2.13) ein, die distale Stift (1.8) jedes Oberschenkelarmes (1.1, 1.2) fügt sich in die zweite Randöffnung (2.6) jedes Unterschenkelarmes (2.1, 2.2, 2.13) ein, die distale Stift (2.7) jedes Unterschenkelarmes (2.1, 2.2, 2.13) fügt sich in die erste Öffnung (1.9) jedes Unterschenkelarmes (1.1, 1.2) ein und die proximale Stift (2.8) jedes Unterschenkelarmes (2.1, 2.2, 2.13) fügt sich in die zweite Öffnung (1.10) jedes Unterschenkelarmes (1.1, 1.2) ein; diese Stifte (1.5, 1.6, 1.7, 1.8) und die Öffnungen (1.9, 1.10), die sich auf dem abgerundeten distalen Ende des seitlichen Oberschenkelarmes (1.1) befinden, und die Öffnungen (2.4, 2.5, 2.6) sowie die Stiften (2.7, 2.8), die sich auf dem abgerundeten proximalen Ende des seitlichen Unterschenkelarmes (2.1) bilden ein seitliches Gelenk der Vorrichtung; analog bilden die Stifte (1.5, 1.6, 1.7, 1.8) und die Öffnungen (1.9, 1.10), die sich auf dem abgerundeten distalen Ende des medialen Oberschenkelarmes (1.2) befinden, und die Öffnungen (2.4, 2.5, 2.6) sowie die Stiften (2.7, 2.8), die sich auf dem abgerundeten proximalen Ende des medialen Unterschenkelarmes (2.2, 2.13) ein mediales Gelenk der Vorrichtung; die Stifte (1.5, 1.6, 1.7, 1.8) der distalen Enden der Oberschenkelarme (1.1, 1.2) sind mit einer externen Platte (4) mit jedem Unterschenkelarm (2.1, 2.2, 2.13) durch Befestigungselemente verbunden; jede Platte (4) weist koaxiale Bohrungen mit den Stiften (1.5, 1.6, 1.7, 1.8) jedes Oberschenkelarm (1.1, 1.2) auf; durch diese Bohrungen der Platte (4) verlaufen die freien Enden derselben Stiften (1.5, 1.6, 1.7, 1.8) oder die oben genannten Befestigungselemente, die sich in den Stiften (1.5, 1.6, 1.7, 1.8) einfügen und dabei jede Platte (4) auf jedem Oberschenkelarm (1.1, 1.2) blockieren sowie die Trennung vom entsprechenden Unterschenkelarm (2.1, 2.2, 2.13) verhindern; die Höhe dieser Stiften (1.5, 1.6, 1.7, 1.8) übersteigt die Stärke jedes Unterschenkelarmes (2.1, 2.2, 2. 13), so dass dieser sich zwischen der Platte (4) und dem Oberschenkelarm (1.1, 1.2) bewegen kann.

2. Ergonomische Knieausrüstung, laut Patentanspruch 1, **gekennzeichnet dadurch, dass** ein bogenförmiges Unterschenkelband (2.11) aus hartem Material, mit einer guten elastischen Reaktion als Federstahl, das seitliche Unterschenkelarm (2.1) und das mediale Unterschenkelarm miteinander verbindet; dieses Unterschenkelband (2), die sich zentral auf dem Unterschenkelhalter (2) befindet, umläuft den Vorderteil des Beins; die Symmetrieachse des Unterschenkelbandes (2.11) ist zur Symmetrieachse des Oberschenkelbandes (1.3) parallel; das mediale Unterschenkelarm ist in zwei Teilen geteilt: einem medialen festen Unterschenkelarm (2.12), mit dem Unterschenkelband (2.11) verbunden, und einem beweglichen medialen Unterschenkelarm (2.13), in dem der mediale feste Unterschenkelarm (2.12) mittels einem medialen Gelenk verzapft; der freie mediale Unterschenkalarm (2.13) besteht aus einem geraden Band, der sich zwischen dem mediale Oberschenkelarm (1.2) und dem festen medialen Unterschenkelarm (2.12) befindet; das feste mediale Oberschenkelarm (1.2) des Oberschenkelhalters (1) ist mit dem proximalen Ende des freien medialen Unterschenkelarm (2.13) mittels einem medialen Gelenk befestigt, das aus den Stiften (1.5, 1.6, 1.7, 1.8) besteht, und der Öffnungen (1.9, 1.10), die sich auf dem abgerundeten distalen Ende des medialen Oberschenkelarmes (1.2) befinden, sowie der Öffnungen (2.4, 2.5, 2.6) und Stiften (2.7, 2.8), die sich auf dem abgerundeten proximalen Ende des freien medialen Unterschenkelarmes (2.13); das distale Ende des freien medialen Armes (2.13) ist abgerundet und weist eine Bohrung (2.14) auf, die sich auf der Symmetrieachse desselben freien, medialen Armes (2.13) befindet; in dieser Bohrung verzapft sich eine Stift (2.15), die sich auf der Kreuzung zwischen der Symmetrieachse des Unterschenkelbands (2.11) und der Symmetrieachse des freien medialen Unterschenkelarms (2.12) befindet; auf der Symmetrieachse des freien medialen Unterschenkelarmes (2.13), proximal zur Bohrung (2.14), befindet sich eine kreisbogenförmige Öffnung (2.16), die abgerundete Enden aufweist; das Zentrum eines Endes der Öffnung (2.16) befindet sich auf der Symmetrieachse des freien medialen Unterschenkelarmes (2.13); die Öffnung (2.16) entwickelt sich in Richtung des vorderen Knies; in diese Öffnung (2.16) wird eine zweite Stift (2.17) eingesteckt, die auf dem festen medialen Unterschenkelarm (2.12) befestigt ist und proximal zur Stift (2.15) auf der Symmetrieachse desselben festen medialen Unterschenkelarmes (2.12) steht; in diese Öffnung (2.16) wird eine zweite Stift (2.17) eingesteckt, die auf dem festen medialen Unterschenkelarm (2.12) befestigt ist und proximal zur Stift (2.15) auf der Symmetrieachse desselben festen medialen Unterschenkelarmes (02:13:00) steht; diese Stifte (2.15, 2.17) des festen medialen Unterschenkelarmes (2.12) sind mit einer externen Platte (5) durch Befestigungselemente verbunden; die Platte (5) weist koaxiale Bohrungen mit den Stiften (2.15, 2.17) auf; durch diese Bohrungen der Platte (5) verlaufen die freien Enden derselben Stiften (2.15, 2.17) oder die oben genannten Befestigungselemente, die sich in den Stiften (2.15, 2.17) einfügen und dabei die Platte (5) auf dem festen medialen Unterschenkelarm (2.13) blockieren sowie die Trennung des freien medialen Unterschenkelarms (2.13) vom festen medialen Unterschenkelarm (2.12) verhindern; die Höhe der oben genannten Stifte (2.15, 2.17) übersteigt die Stärke des freien medialen Unterschenkelarmes (2.13), so dass dieser sich zwischen der Platte (5) und dem Unterschenkelarm (2.13) bewegen kann, das mediale Oberschenkelarm (1.2) ist etwas länger als das seitliche Oberschenkelarm (1.1).

3. Ergonomische Knieausrüstung, laut Patentanspruch 2, **gekennzeichnet dadurch, dass** der kreisförmige Teil der sich auf dem freien medialen Unterschenkelarm (2.13) befindlichen Randöffnung (2.5) eine Weite von ca. 15°-20° aufweist, während der kreisförmige Teil der sich auf dem seitlichen Unterschenkelarm (2.1) befindlichen Öffnung (2.5) eine Weite von ca. 25°-30° aufweist; der spiralförmige Teil der Randöffnung (2.5) des freien medialen Unterschenkelarmes (2.13) erstreckt sich für ca. 115°-120°, während der Teil der sich auf dem seitlichen Unterschenkelarm (2.1) befindlichen Randöffnung (2.5) für ca. 105°-110° erstreckt; der spiralförmige Teil der Randöffnung (2.5) des seitlichen Unterschenkelarmes (2.1) rückt gegen das Zentrum der Platte mehr als der spiralförmige Teil der Randöffnung (2.5) des freien medialen Unterschenkelarmes (2.13) ein; der kreisförmige Teil der sich auf dem freien medialen Unterschenkelarm (2.13) befindlichen Randöffnung (2.6) weist eine Weite von ca. 15°-20° auf, während der kreisförmige Teil der sich auf dem seitlichen Unterschenkelarm (2.1) befindlichen Öffnung (2.6) eine Weite von ca. 25°-30° aufweist; der spiralförmige Teil der Randöffnung (2.6) des freien medialen Unterschenkelarmes (2.13) erstreckt sich für ca. 115°-120°, während der Teil der sich auf dem seitlichen Unterschenkelarm (2.1) befindlichen Randöffnung (2.6) für ca. 105°-110° erstreckt; der spiralförmige Teil der Randöffnung (2,6) des seitlichen Unterschenkelarmes (2.1) rückt gegen das Zentrum der Platte mehr als der spiralförmige Teil der Randöffnung (2.6) des freien medialen Unterschenkelarmes (2.13) ein; die Öffnung (2.4) des seitlichen Unterschenkelarms (2.1) ist länger als die Öffnung des freien medialen Unterschenkelarms (2.13); der kreisförmige Teil der sich auf dem seitlichen Oberschenkelarm (1.1) befindlichen Öffnung (1.9) weist eine Weite von ca. 25°-30° auf, während der kreisförmige Teil der sich auf dem medialen Oberschenkelarm (1.2) befindlichen Öffnung (1.9) eine Weite von ca. 15°-20° aufweist; der spiralförmige Teil der sich auf dem seitlichen Oberschenkelarm (1.1) befindlichen Öffnung (1.9) erstreckt sich für ca. 105°-110°, während der spiralförmige Teil der sich auf dem medialen Oberschenkelarm (1.2) befindlichen Öffnung (1.9) sich für ca. 115°-120° erstreckt; der spiralförmige Teil der Randöffnung (1.9) des seitlichen Oberschenkelarmes (1.1) rückt gegen das Zentrum der Platte mehr als der spiralförmige Teil der Randöffnung (1.9) des medialen Oberschenkelarmes (1.2) ein; der kreisförmige Teil der sich auf dem seitlichen Oberschenkelarm (1.1) befindlichen Öffnung (1.10) weist eine Weite von ca. 25°-30° auf, während der kreisförmige Teil der sich auf dem medialen Oberschenkelarm (1.2) befindlichen Öffnung (1.10) eine Weite von ca. 15°-20° aufweist; der spiralförmige Teil der sich auf dem seitlichen Oberschenkelarm (1.1) befindlichen Öffnung (1.10) erstreckt sich für ca. 105°-110°, während der spiralförmige Teil der sich auf dem medialen Oberschenkelarm (1.2) befindlichen Öffnung (1.10) sich für ca. 115°-120° erstreckt; der spiralförmige Teil der Randöffnung (1.10) des seitlichen Oberschenkelarmes (1.1) rückt gegen das Zentrum der Platte weniger als der spiralförmige Teil der Randöffnung (1.10) des medialen Oberschenkelarmes (1.2) ein; der sich am Ende des freien medialen Unterschenkelarms (2.13) befindliche kreisbogenförmige peripherische Rand (2.9) weist eine Weite von ca. 15°-20° auf, während der entsprechende Rand auf dem seitlichen Unterschenkelarm (2.1) eine Weite von ca. 25°-30° aufweist; der sich am Ende des freien medialen Unterschenkelarm (2.13) befindliche peripherische Rand (2.10) weist eine Weite von ca. 115°-120° auf, während der entsprechende Rand auf dem seitlichen Unterschenkelarm (2.1) eine Weite von ca. 105°-110° aufweist; der spiralförmige Teil der Randöffnung (2.10) des freien medialen Unterschenkelarmes (2.13) rückt gegen das Zentrum der Platte mehr als der spiralförmige Teil der Randöffnung (2.10) des seitlichen Unterschenkelarmes (2.1) ein.

4. Ergonomische Knieausrüstung, laut Patentansprüche 1 und 2, **gekennzeichnet dadurch, dass** die Stifte (1.5, 1.6, 1.7, 1.8) der distalen Enden der Oberschenkelarme (1.1, 1.2) und die Stifte (2.15, 2.17) des festen medialen Unterschenkelarmes (2.12) entlang der eigenen Längsachse gelocht sind und dass jedes Loch ein Gewindeloch ist; durch diese Löcher der Platten (4, 5) werden Schrauben eingefügt, die sich in den Gewindelöcher der Stifte (1.5, 1.6, 1.7, 1.8, 2.15, 2.17) einklemmen.

5. Ergonomische Knieausrüstung, laut Patentansprüche 1 und 2, **gekennzeichnet dadurch, dass** die Stifte (1.5, 1.6, 1.7, 1.8) der distalen Enden der Oberschenkelarme (1.1, 1.2) und die Stifte (2.15, 2.17) des festen medialen Unterschenkelarmes (2.12) entlang der eigenen Längsachse gelocht sind; durch diese Löcher der Platten (4, 5) werden Nieten eingefügt, derer freie Ende sich in den Löcher der Stifte (1.5, 1.6, 1.7, 1.8, 2.15, 2.17) einklemmt.

6. Ergonomische Knieausrüstung, laut Patentansprüche 1 und 2, **gekennzeichnet dadurch, dass** die Stifte (1.5, 1.6, 1.7, 1.8) der distalen Enden der Oberschenkelarme (1.1, 1.2) und die Stifte (2.15, 2.17) des festen medialen Unterschenkelarmes (2.12) am jeweiligen Ende ein Außengewinde aufweisen; auf jedem solchen Gewinde klemmt ein Bolzen ein.

7. Ergonomische Knieausrüstung, laut Patentanspruch 1, **gekennzeichnet dadurch, dass** oben genannte Stifte (1.5, 1.6, 1.7, 1,8, 2.15, 2.17) der Oberschenkelarme (1.1, 1.2) und der medialen Unterschenkelarme (2.1, 2.2, 2.13) bei spezifischen Fehlern entfernt werden können.

8. Ergonomische Knieausrüstung, laut Patentanspruch 2, **gekennzeichnet dadurch, dass** das Unterschenkelband (2.11) mit jedem Unterschenkelarm (2.2, 2.3) mittels einem Gelenk verbunden werden kann.

9. Ergonomische Knieausrüstung, laut Patentanspruch 2, **gekennzeichnet dadurch, dass** das distale Ende des freien medialen Unterschenkelarmes (2.13) mehrere Löcher (2.18) entlang seiner Symmetrieachse und mehrere kreisbogenförmigen Öffnungen aufweist; das Zentrum eines halbrunden Endes der Öffnungen (2.19) befindet sich entlang derselben Symmetrieachse.

10. Ergonomische Knieausrüstung, laut Patentanspruch 2, **gekennzeichnet dadurch, dass** der seitliche Unterschenkelarm in einem proximalen seitlichen Unterschenkelarm (2.20) und einem distalen seitlichen Unterschenkelarm (2.21) geteilt ist, wobei der proximale seitliche Unterschenkelarm (2.20) länger ist und der distale seitliche Unterschenkelarm (2.21) mit dem Unterschenkelband (2.11) befestigt ist; auf der Verlängerung des proximalen seitlichen Unterschenkelarm (2.20) und entlang seiner Symmetrieachse sind mindestens drei gleichabständigen Löcher (2.22) vorhanden; auf dem distalen seitlichen Unterschenkelarm (2.21) sind mindestens zwei Gewindelöcher (2.23) vorhanden, koaxial zu min. zwei Löcher (2.22), die die sich auf dem proximalen seitlichen Unterschenkelarm (2.20) befinden; zwei Schrauben verlaufen durch die Löcher (2.22) des proximalen seitlichen Unterschenkelarm (2.20) und verbinden sich mit den zwei Gewindelöcher (2.23) auf dem distalen seitlichen Unterschenkelarm (2.21).

## Revendications

1. Dispositif ergonomique de genou pour résoudre les charges verticales, formé d'un support fémoral (1) et d'un support tibial (2), le dit support fémoral (1) étant formé d'un bras fémoral (1.1) latéral et d'un bras fémoral (1.2) médial, parallèles entre eux et à l'axe longitudinal de la cuisse unis entre eux moyennant des engins réglables en longueur aptes à bloquer le support fémoral(1) sur la cuisse et le dit support tibial (2) étant formé d'un bras tibial (2.1) latéral et au moins d'un bras tibial (2.2, 2.12,2.13) médial, parallèles entre eux et à l'axe longitudinal de la jambe proprement dite; les susdits bras fémoraux (1.2,1.2) et tibiaux (2.2, 2.12) sont bloqués, respectivement, sur la cuisse et sur la jambe proprement dite au moyen de sangles (3) présentant une face en nylon velcro®, passant aux travers des ouvertures linéaires (1.4, 2.3) réalisées sur les bras fémoraux (1.1, 1.2) et tibiaux (2.1, 2.2) et une lame (1.3, 2.11); la déjà nommée lame (1.3) en matière rigide ou semi-rigide façonnée à arc contourne avec sa superficie interne la partie antérieure de la cuisse; les extrémités distales des bras fémoraux (1.1, 1.2) et les extrémités proximales des bras tibiaux (2.1, 2.2, 2.13) sont articulées entre elles moyennant au moins deux joints l'un positionné latéralement et l'autre médialement au genou; les extrémités distales de chacun bras fémoraux (1.1, 1.2) et les extrémités proximales de chacun des bras tibiaux (2.1, 2.2, 2.13) formant les joints sont façonnées à plaque set arrondies; le susdit dispositif se caractérise parce que sur l'extrémité distale arrondie de chacun bras fémoraux (1.1, 1.2) sont fixés quatre pivots (1.5, 1.6, 1.7, 1.8) et sont réalisées deux ouvertures (1.9, 1.10); un premier pivot (1.5) est placé à l'extrémité distale de chacun des bras fémoraux (1.1, 1.2) en position centrale le long d'un axe «a» qui coïncide avec l'axe de symétrie longitudinal du bras fémoral (1.1, 1.2) même; un deuxième pivot (1.6) est placé sur l'extrémité distale de chacun des bras fémoraux (1.1, 1.2) en position périphérique et à une distance «I» par rapport au pivot (1.5) central, le long d'un axe «b» orthogonal à l'axe «a»; l'axe «a» et l'axe «b» divisent idéalement l'extrémité distale du bras fémoral (1,1) latéral et du bras fémoral (1.2) médial en quatre cadrans, le premier orienté vers le pied et la partie postérieure de la jambe, le second orienté vers le pied et la partie antérieure de la jambe, le troisième disposé vers la hanche du membre inférieure et la partie postérieure de la jambe et le quatrième disposé vers la hanche du membre inférieure et la partie antérieure de la jambe; un troisième pivot (1.7) est placé en position proximale par rapport aux déjà cités pivots (1.5, 1.6) et dans le cadran placé vers la hanche du membre inférieure et la partie postérieure de la jambe; le dit pivot (1.7) proximal est positionné à une distance «r» par rapport au pivot (1.5) central; l'axe «d» passant par le centre du pivot (1.7) proximal et par le centre du pivot (1.5) central diverge de quelques degrés de l'axe «a» et le bord périphérique du pivot (1.7) proximal est tangent au même axe «a»; le susmentionné quatrième pivot (1.8) est placé dans le cadran orienté vers le pied et la partie antérieure de la jambe, sur le prolongement du déjà cité axe «d» à une distance «r» par rapport au pivot (1.5) central; le bord périphérique du pivot (1.8) distal est tangent à l'axe «a» et localisé en position diamétralement opposée au pivot (1.7) proximal par rapport au pivot (1.5) central; la susnommée première ouverture (1.9) est placée en proximité du bord périphérique de l'extrémité distale arrondie de chacun des bras fémoraux (1.1, 1.2) et se développe dans les deux cadrans disposés vers la partie postérieure de la jambe pour 130°-140°; le centre d'une première extrémité semi-circulaire de l'ouverture (1.9) est placé à une distance «r» du pivot (1.5) central le long d'un axe «d1» spéculaire à l'axe «d» par rapport à l'axe «a»; le bord de la première extrémité semi-circulaire de la déjà nommée première ouverture (1.9) est tangent à l'axe «a» et au bord périphérique du pivot (1.8) distal; en partant de la susmentionnée première extrémité pour les premier 25°-30° la déjà citée ouverture (1.9) est façonnée à arc de circonférence; à partir des 25°-30° jusqu'aux 130°-140° la suscitée première Ouverture (1.9) est façonnée à spirale qui se rapproche au centre de l'extrémité distale arrondie du bras fémoral (1.1) latéral et du bras fémoral (1.2) médial; la susnommée deuxième ouverture (1.10) est placée en position diamétralement opposée à la suscité première ouverture (1.9) par rapport à l'axe «a» et dans les deux cadrans disposé vers la partie antérieure de la jambe; le centre d'une des premières extrémités de la deuxième ouverture (1.10) est positionné à une distance «r» du pivot (1.5) central le long du susdit axe «d1»; le bord de la première extrémité semi-circulaire de la susdite seconde ouverture (1.10) est tangent à l'axe «a» et au bord périphérique du pivot (1.7) proximal; en partant depuis la susdite première extrémité semi-circulaire pour les premier 25°-30°, la susdite seconde ouverture (1.10) est façonnée à arc de circonférence; la suscité deuxième ouverture (1.10) à partir des 25°-30° jusqu'aux 130°-140° est façonnée à spirale qui, différemment de la première ouverture (1.9), s'éloigne du centre de l'extrémité distale arrondie du bras fémoral (1.1, 1.2); dans la plaque qui se trouve à l'extrémité proximale de chacun des bras tibiaux (2.1, 2.2, 2.13) ont été réalisées trois ouvertures (2.4, 2.5, 2.6) et sur la même plaque sont fixés deux pivots (2.7,2.8); la première ouverture (2.4) tibial a une forme rectangulaire avec les extrémités arrondies et qui a été réalisée faisant un trou au centre de la plaque de l'extrémité proximale du bras tibial (2.1,2.2, 2.13) médial et en se déplaçant vers le pied le long d'un axe «a1» de symétrie longitudinale du bras tibial (2.1, 2.2, 2.13); la deuxième ouverture (2.5) s'étend pour environ 130°-140° et est placé en position périphérique sur l'extrémité proximale de chacun des bras tibiaux (2.1, 2.2, 2.13); une première extrémité de telle deuxième ouverture (2.5) périphérique est située sur un axe «b1», orthogonal à l'axe «a1 », à une distance «I» du centre du suscité trou central duquel prend origine la première ouverture (2.4); l'autre extrémité de la deuxième ouverture (2.5) tibiale est placée à 130°-140° par rapport à l'axe «b1»; en partant du susdit axe «b1» pour les premiers 25°-30° la susnommée deuxième ouverture (2.5) tibiale est façonnée à arc de circonférence ayant le propre centre coïncidant avec celui du trou générant l'ouverture centrale (2.4) tibiale et un rayon égal à «I»; depuis les 25°-30° jusqu'aux restants 105°-110° c'est une spirale rentrante vers le centre de l'extrémité proximale de chacun des bras tibiaux (2.1, 2.2, 2.13); l'axe longitudinal de la susnommée spirale est obtenu de la séquence des points de l'extrémité d'un segment de longueur «I» dont la deuxième extrémité se meut le long de l'axe longitudinal de l'ouverture (2.4) centrale en se déplaçant du centre de chacun des bras tibiaux (2.1, 2.2, 2.13) vers la périphérie; le susdit axe «a1» et le susdit axe «b1» divisent idéalement l'extrémité proximale de chacun des bras tibiaux (2.1, 2.2, 2.13) en quatre cadrans, le premier disposé vers le pied et la partie postérieure de la jambe, le deuxième orienté vers le pied et la partie antérieure de la jambe, le troisième orienté vers la hanche du membre inférieur et la partie postérieure de la jambe et le quatrième disposé vers la hanche du membre inférieur et la partie antérieure de la jambe; la susdite ouverture (2.4) centrale tibiale est positionnée à l'intérieur des deux cadrans orientés vers le pied tandis que la susdite deuxième ouverture (2.5) tibiale est positionnée à l'intérieur des deux cadrans orientés vers la hanche du membre inférieur; le centre d'une première extrémité semi-circulaire de la troisième ouverture (2.6) est placé à une distance «r» à partir de l'intersection des axes «a1», «b1» à l'intérieur du cadran disposé vers le pied et la partie antérieure de la jambe et diverge de quelques degré de l'axe «a1»; le bord de l'extrémité de la susmentionnée troisième ouverture (2.6) tibiale est tangent à l'axe «a1»; la susnommée troisième ouverture (2.6) tibiale s'étend pour environ 130°-140° et c'est un arc de circonférence pour les premiers 25°-30° tandis que à partir des 25°-30° jusqu'aux 130°-140° c'est une spirale qui se rapproche au centre de l'extrémité proximale arrondie de chacun des bras tibiaux (2.1, 2.2, 2.13); le premier pivot (2.7) tibial est placé en position distale par rapport à l'ouverture (2.4) centrale et à une distance «r» de l'intersection des axes «a1», «b1» dans le cadran disposé vers le pied et la partie postérieure de la jambe; l'axe «d2» passant par le centre du pivot (2.7) et l'intersection des axes «a1», «b1» diverge de quelques degrés de l'axe «a1»; le bord périphérique du premier pivot (2.7) tibial est tangent à l'axe «a1» même et à la première extrémité de la troisième ouverture tibiale (2.6); le susnommé axe «d2» se prolonge vers le bord périphérique proximal du bras tibial (2.2) médial; sur tel axe, à une distance «r» par rapport à l'intersection des axes «a1 », «b1», est placé un second pivot (2.8) tibial; le dit second pivot (2.8) tibial est placé en position diamétralement opposé au premier pivot (2.7) tibial par rapport à l'axe «a1» et par conséquent l'axe «d2» passant par le centre du pivot (2.8) et l'intersection des axe «a1», «b1» diverge de quelques degrés de l'axe «a1» mais en direction opposée; de même le bord périphérique du deuxième pivot (2.8) tibial est tangent à l'axe «a»; le susdit deuxième pivot (2.8) tibial proximal est positionné dans le cadran orienté vers la hanche du membre inférieur et la partie antérieure de la jambe; une extrémité des deux ouvertures périphériques (2.5) des bras tibiaux (2.1, 2.2, 2.13) est située sur l'axe «b1», orthogonal à l'axe «a1»; les deux ouvertures périphériques (2.5) se développent vers la partie antérieure du genou et spéculativement à ce dernier; de même les deux ouvertures périphériques (2.6) des bras tibiaux (2.1, 2.2, 2.13) se développent vers la partie antérieure du genou et spéculativement à ce dernier; de façon analogue les pivots (2.7, 2.8) du bras tibial (2.1) latéral sont spéculaires aux pivots (2.7, 2.8)du bras tibial (2.2, 2.13) médial et les ouvertures (1.9,1.10) et les pivots (1.6, 1.7, 1.8) du bras fémoral (1.1) latéral sont spéculaires aux ouvertures (1.9, 1.10) et les pivots (1.6, 1.7, 1.8) du bras fémoral (1.2) médial; les susdits pivots (1.5, 1.6, 1.7, 1.8) présents sur chacun des bras fémoraux (1.1, 1.2) sont orientés vers l'extrémité proximale arrondie de chacun des bras tibiaux (2.1, 2.2, 2.13), tandis que les pivots (2.7 , 2.8) présents sur chacun des bras tibiaux (2.1, 2.2, 2.13) sont orientés vers l'extrémité distale arrondie de chacun des bras fémoraux (1.1. 1.2); une première partie du bord périphérique (2.9) de l'extrémité de chacun des bras tibiaux (2.1, 2.2, 2.13), qui se développe dans le cadran postérieure de la jambe orienté vers la hanche du membre, est façonnée à arc de cercle; dans une seconde partie, qui se développe dans le cadran postérieure orienté vers le pied, le bord périphérique (2.10), est façonné en spirale qui tend à s'éloigner par rapport au centre de l'extrémité di chacun des bras tibiaux (2.1, 2.2,2.13); la superficie périphérique du susdit pivot (1.7) proximale de chacun des bras fémoraux (1.1, 1.2)reste toujours en contact avec le bord périphérique (2.9, 2.10) de chacun des bras tibiaux (2.1, 2.2, 2.13) au cours du mouvement de roto-translation de ce dernier par rapport à chacun des bras fémoraux (1.1,1.2); le pivot (1.5) central de chacun des bras fémoraux (1.1, 1.2) va se loger dans l'ouverture (2.4) centrale de chacun des bras tibiaux (2.1, 2.2, 2.13), le deuxième pivot (1.6) de chacun des bras fémoraux (1.1, 1.2) vase loger dans la première ouverture (2.5) périphérique de chacun des bras tibiaux (2.1, 2.2, 2.13), le pivot (1.8) distal de chacun des bras fémoraux (1.1, 1.2) va se loger dans la seconde ouverture (2.6) périphérique de chacun des bras tibiaux (2.1, 2.2, 2.13), le pivot (2.7) distal de chacun des bras tibiaux (2.1, 2.2, 2.13) va se loger dans la première ouverture (1.9) de chacun des bras fémoraux (1.1, 1.2) et le pivot (2.8)proximal de chacun des bras tibiaux (2.1, 2.2, 2.13) va se loger dans la seconde ouverture (1.10) de chacun des bras fémoraux (1.1, 1.2); les pivots (1.5, 1.6, 1.7, 1.8) et les ouvertures (1.9, 1.10) présents sur l'extrémité distale arrondie du bras fémoral (1.1) latéral et les ouvertures (2.4, 2.5, 2.6) et les pivots (2.7, 2.8) présents sur l'extrémité proximale arrondie du bras tibial (2.1) latéral forment un joint latéral du dispositif; de façon analogue les pivots (1.5, 1.6, 1.7, 1.8) et les ouvertures (1.9, 1.10) présents sur l'extrémité distale arrondie du bras fémoral (1.2) médial, les ouvertures (2.4, 2.5, 2.6) et les pivots (2.7, 2.8) présents sur l'extrémité proximale arrondie du bras tibial (2.2, 2.13) médial forment un joint médial du dispositif; les susdits pivots (1.5, 1.6, 1.7, 1.8) des extrémité distales des bras fémoraux (1.1, 1.2) sont unis à une plaque (4) externe à chacun des bras tibiaux (2.1, 2.2, 2.13) au moyen d'organes de fixage; chaque plaque (4) présente des trous coaxiaux avec les pivots (1.5, 1.6, 1.7,1.8) de chacun des bras fémoraux (1.1, 1.2); au travers des susnommés trous de la plaque (4) passent les extrémités libres des pivots (1.5, 1.6, 1.7, 1.8) eux-mêmes ou les suscités organes de fixage qui vont s'engager sur les pivots (1.5, 1.6, 1.7, 1.8), bloquant chaque plaque (4) sur chacun des bras fémoraux (1.1, 1.2) et empêchant sa séparation du respectif bras tibial (2.1, 2.2, 2.13);la hauteur des susdits pivots (1.5, 1.6, 1.7, 1.8) est supérieure à l'épaisseur de chacun des bras tibiaux (2.1, 2.2, 2.13) de façon que ce-dernier puisse se mouvoir librement entre la plaque (4) et le bras fémoral (1.1, 1.2).

2. Dispositif ergonomique pour le genou, de par la revendication 1, **caractérisé** du fait qu'une lame tibiale (2.11) façonnée à arc, en matière rigide avec une bonne réponse harmonique tel que l'acier harmonique, unit entre eux le bras tibial (2.1) latéral et le bras tibial médial; la susdite lame tibiale (2.11), placée en position centrale au support tibial (2), contourne la partie antérieure de la jambe proprement dite; l'axe de symétrie de la lame tibiale (2.11) est parallèle à l'axe de symétrie de la lame fémorale (1.3); le bras tibial médial est séparé en deux parties: un bras tibial (2.12) médial fixe unit à la lame tibiale (2.11) et un bras tibial (2.13) médial mobile pivotant sur bras tibial (2.12) médial fixe moyennant un second joint médial; le bras tibial (2.13) médial libre est constitué d'une lame rectiligne qui s'étend entre le bras fémoral (1.2) médial et le bras tibial (2.12) médial fixe; le bras fémoral (1.2) médial fixe du support fémoral (1) est connexe à l'extrémité proximale du bras tibial (2.13) médial libre moyennant un joint médial formé des pivots (1.5, 1.6, 1.7, 1.8) et les ouvertures (1.9, 1.10) présentes sur l'extrémité distale arrondie du bras fémoral (1.2) médial et les ouvertures (2.4, 2.5, 2.6) et les pivots (2.7, 2.8) présents sur l'extrémité proximale arrondie du bras tibial (2.13) médial libre; l'extrémité distale du bras (2.13) médial libre est arrondie et présente un trou (2.14) obtenu sur l'axe de symétrie du bras (2.13) médial lui-même libre, dans lequel va se loger un pivot (2.15) placé sur l'intersection entre l'axe de symétrie de la lame tibiale (2.11) et l'axe de symétrie du bras tibial (2.12) médial fixe; sur l'axe de symétrie du bras tibial (2.13) médial libre, en position proximale par rapport au trou (2.14), est placé une ouverture (2.16) à arc de cercle, qui a les extrémités arrondies; le centre d'une des extrémités de l'ouverture (2.16) est placé sur l'axe de symétrie du bras tibial (2.13) médial libre; l'ouverture (2.16) se développe en direction antérieure au genou; dans la susdite ouverture (2.16) est inséré un second pivot (2.17) fixé au bras tibial (2.12) médial fixe et placé en position proximale par rapport au pivot (2.15) sur l'axe de symétrie du bras tibial (2.12) médial fixe lui-même; les deux pivots (2.15, 2.17) fixés au bras tibial (2.12) médial fixe, le trou (2.14) et l'ouverture (2.16) à arc de cercle du bras tibial (2.13) médial libre forment un second joint médial; les susdits pivots (2.15,2.17) du bras tibial (2.12) médial fixe sont unis à une plaque (5) extérieure à l'aide d'organes de fixage; la plaque (5) présente des trous coaxiaux avec les pivots (2.15, 2.17); au travers des susdits trous de la plaque (5) passent les extrémités libres des pivots (2.15, 2.17) eux-mêmes ou les déjà mentionnés organes de fixage qui vont s'engager sur les pivots (2.5, 2.7), bloquant la plaque (5) sur le bras tibial (2.12) médial fixe et empêchant le détachement du bras tibial (2.13) médial libre du bras tibial (2.12) médial fixe; la hauteur des susmentionnés pivots (2.15, 2.17) est supérieure à l'épaisseur du bras tibial (2.13) médial libre de façon que ce dernier puisse se mouvoir librement entre la plaque (5) et le bras tibial (2.12) médial fixe; le bras fémoral (1.2) médial est légèrement plus long du bras fémoral (1.1) latéral.

3. Dispositif ergonomique pour le genou, à partir de la revendication 2, **caractérisé** du fait que la partie circulaire de l'ouverture périphérique (2.5) située sur le bras tibial (2.13) médial libre a une ampleur d'environ 15°-20°, tandis que la partie circulaire de l'ouverture (2.5) située sur le bras tibial (2.1) latérale a une ampleur d'environ 25°-30°; la partie à spirale de l'ouverture périphérique (2.5) du bras tibial (2.13) médial libre s'étend pour environ 115°-120°, tandis que celle de l'ouverture périphérique (2.5) située sur le bras tibial (2.1) latéral s'étend pour environ 105°-110°; la partie à spirale de l'ouverture périphérique (2.5) du bras tibial (2.1) latéral rentre vers le centre de la plaque de façon majeure par rapport à la partie à spirale de l'ouverture périphérique (2.5) du bras tibial (2.13) médial libre; la partie circulaire de l'ouverture périphérique (2.6) située sur le bras tibial (2.13) médial libre a une ampleur d'environ 15°-20°, tandis que la partie circulaire de l'ouverture (2.6) située sur le bras tibial (2.1) latéral a une ampleur d'environ 25°-30°; la partie à spirale de l'ouverture périphérique (2.6) du bras tibial (2.13) médial libre s'étend pour environ 115°- 120°, tandis que celle de l'ouverture périphérique (2.6) située sur le bras tibial (2.1) latéral s'étend pour environ 105°- 110°; la partie à spirale de l'ouverture périphérique (2.6) du bras tibial (2.1) latéral rentre vers le centre de la plaque de façon majeure par rapport à la partie à spirale de l'ouverture périphérique (2.6) du bras tibial (2.13) médial libre ; l'ouverture (2.4) du bras tibial (2.1) latéral est plus allongée de l'ouverture du bras tibial (2.13) médial libre; la partie circulaire de l'ouverture (1.9) située sur le bras fémoral (1.1) latéral a une ampleur d'environ 25°-30°, tandis que la partie circulaire de l'ouverture (1.9) située sur le bras fémoral (1.2) médial a une ampleur d'environ 15°-20° ; la partie à spirale de l'ouverture (1.9) située sur le bras fémoral (1.1)latéral s'étend pour environ 105°-110°, tandis que la partie en spirale de l'ouverture (1.9) située sur le bras fémoral (1.2) médial s'étend pour environ 115°-120°; la partie à spirale de l'ouverture périphérique (1.9) du bras fémoral (1.1) latéral rentre vers le centre de la plaque d'une manière supérieure par rapport à la partie à spirale de l'ouverture périphérique (1.9) du bras fémoral (1.2) médial; la partie circulaire de l'ouverture (1.10) située sur le bras fémoral (1.1) latéral a une ampleur d'environ 25°-30°, tandis que la partie circulaire de l'ouverture (1.10) située sur le bras fémoral (1.2) médial a une ampleur d'environ 15°-20°; la partie à spirale de l'ouverture (1.10) située sur le bras fémoral (1.1) latéral s'étend pour environ 105°-110°, tandis que la partie à spirale de l'ouverture (1.10) située sur le bras fémoral (1.2) médial s'étend pour environ 115°-120°; la partie à spirale de l'ouverture périphérique (1.10) du bras fémoral (1.1) latéral rentre vers le centre de la plaque de façon mineure par rapport à la partie à spirale de l'ouverture périphérique (1.10) du bras fémoral (1.2) médial; le bord périphérique (2.9) à arc de cercle présent à l'extrémité du bras tibial (2.13) médial libre a une ampleur de 15-20°, tandis que celui qui est présent sur le bras tibial (2.1) latéral a une ampleur de 25-30°; le bord périphérique (2.10)à spirale présent à l'extrémité du bras tibial (2.13) médial libre a une ampleur de 115°-120°, tandis que celui qui est présent sur le bras tibial (2.1) latéral a une ampleur de 105°-110°; la partie à spirale du bord périphérique (2.10) située sur le bras tibial (2.13) médial libre rentre de façon majeure vers le centre de la plaque même par rapport à la partie à spirale du bord périphérique (2.10) située sur le bras tibial (2.1) latéral.

4. Dispositif ergonomique pour le genou, d'après les revendications 1 et 2 **caractérisé** du fait que les pivots (1.5, 1.6, 1.7, 1.8) des extrémités distales des bras fémoraux (1.1, 1.2) et des pivots (2.15, 2.17) du bras tibial (2.12) médial fixe sont percé le long du propre axe longitudinal et chaque perçage est taraudée; au travers des susdites perforations des plaques (4, 5) passent des vis qui vont se fixer dans les perforations taraudés des pivots (1.5, 1.6, 1.7, 1.8, 2.15, 2.17).

5. Dispositif ergonomique pour le genou, selon les revendications 1 e 2, **caractérisé** du fait que les pivots (1.5, 1.6, 1.7, 1.8) des extrémités distales des bras fémoraux (1.1, 1.2) et les pivots (2.15, 2.17) du bras tibial (2.12) médial fixe sont forés le long du propre axe longitudinal; au travers de perçages des plaques (4, 5) passent des rivets dont l'extrémité libre va se fixer dans les trous des pivots (1.5, 1.6, 1.7, 1.8, 2.15, 2.17).

6. Dispositif ergonomique pour le genou, selon les revendications 1 e 2, **caractérisé** du fait que les pivots (1.5, 1.6, 1.7, 1.8) des extrémités distales des bras fémoraux (1.1, 1.2) et les pivots (2.15, 2.17) du bas tibial (2.12) médial fixe présentent sur leur extrémité libre un taraudage externe; sur chacun des dits taraudages va s'engager un écrou.

7. Dispositif ergonomique pour le genou, selon la revendication 1, **caractérisé** du fait que les dits pivots (1.5, 1.6, 1.7, 1.8, 2.15, 2.17) des bras fémoraux (1.1, 1.2) et des bras tibiaux (2.1, 2.2, 2.13) médiaux peuvent être enlevés en présence de pathologies spécifiques.

8. Dispositif ergonomique pour le genou, selon la revendication 2, **caractérisé** du fait que la lame tibiale (2.11) peut être assemblée à chacun des bras tibiaux (2.2, 2.3) au moyen d'une charnière.

9. dispositif ergonomique pour le genou, selon la revendication 2, **caractérisé** du fait que sur l'extrémité distale du bras tibial (2.13) médial libre sont réalisés plusieurs trous (2.18) le long de son axe de symétrie et plusieurs ouvertures (2.19) à arc de cercle; le centre d'une extrémité semi-circulaire des ouvertures (2.19) est placé le long du même axe de symétrie.

10. Dispositif ergonomique pour le genou, selon la revendication 2, **caractérisé** du fait que le bras tibial latéral est divisé en deux partie: un bras tibial (2.20) latéral proximal et un bras tibial (2.21) latéral distal, dans la présente situation le bras tibial (2.20) latéral proximal est allongé et le bras tibial (2,21) latéral distale est fixé à la lame tibial (2.11); sur le prolongement du bras tibial (2.20) latéral proximal et le long de son axe de symétrie sont réalisés au moins trois trous (2.22) équidistants; sur le bras tibial (2.21) latéral distal, sont réalisés au moins deux trous (2.23) taraudés, coaxiaux à au moins deux trous (2.22) présents sur le bras tibial (2.20) latéral proximal; deux vis traversent les perforations (2.22) du bras tibial (2.20) latéral proximal, et vont s'engager dans deux trous (2.23) taraudés du bras tibial (2.21) latéral distal.
